# EUROPÄISCHE PATENTSCHRIFT

(11) **EP 1 377 569 B1**
(45) Veröffentlichungstag und Bekanntmachung des Hinweises auf die Patenterteilung: **02.11.2005**
(21) Anmeldenummer: 02732498.7
(22) Anmeldetag: 19.03.2002
(51) Int. Cl.: C07D 405/04, C07D 405/14, C07D 213/85, A61K 31/443, A61K 31/4433, A61K 31/444, A61K 31/4439, A61K 31/4418, C07D 417/14

(54) **SUBSTITUIERTE 2-THIO-3,5-DICYANO-4-PHENYL-6-AMINOPYRIDINE MIT ADENOSINREZEPTOR-BINDENDER WIRKUNG UND IHRE VERWENDUNG ALS HERZ-KREISLAUF-MITTEL**
SUBSTITUTED 2-THIO-3,5-DICYANO-4-PHENYL-6-AMINOPYRIDINES WITH ADENOSINE RECEPTOR-BINDING ACTIVITY AND THEIR USE AS CARDIOVASCULAR PREPARATIONS
2-THIO-3,5-DICYANO-4-ARYL-6-AMINOPYRIDINES SUBSTITUEES AYANT UNE ACTIVITE DE LIAISON DE RECEPTEUR ADENOSINE ET UTILISATION EN TANT QU'AGENTS CARDIOVASCULAIRES

(30) Priorität: 30.03.2001 DE 10115922
(43) Veröffentlichungstag der Anmeldung: 07.01.2004
(60) Teilanmeldung: 05011426.3
(73) Patentinhaber: Bayer HealthCare AG, 51368 Leverkusen (DE)
(72) Erfinder: ROSENTRETER, Ulrich, 42349 Wuppertal (DE); KRÄMER, Thomas, 42111 Wuppertal (DE); VAUPEL, Andrea, CH-4125 Riehen (CH); HÜBSCH, Walter, 42113 Wuppertal (DE); DIEDRICHS, Nicole, 42103 Wuppertal (DE); KRAHN, Thomas, 58135 Hagen (DE); DEMBOWSKY, Klaus, Boston, MA 02118 (US); STASCH, Johannes-Peter, 42651 Solingen (DE); SHIMADA, Mitsuyuki, Higashigawa-cho, Nara 630-8323 (JP)
(86) Internationale Anmeldenummer: PCT/EP2002/002998
(87) Internationale Veröffentlichungsnummer: WO 2002/079195

(56) Entgegenhaltungen:
- EP-A- 0 285 267
- EP-A- 0 908 458
- WO-A-01/25210
- WO-A-01/62233
- WO-A-02/06237
- WO-A-94/12493
- WO-A-99/16766
- KANDEEL, EZ-EL-DIN M. ET AL.: "The use of activated double bond systems in heterocyclic syntheses" HETEROCYCLIC COMMUNICATIONS, Bd. 3, Nr. 4, 1997, Seiten 371-380, XP001098754

## Beschreibung

Die vorliegende Erfindung betrifft neue 2-Thio-3,5-dicyano-4-aryl-6-aminopyridine, ein Verfahren zu ihrer Herstellung und ihre Verwendung als Arzneimittel.

Adenosin, ein Nucleosid aus Adenin und D-Ribose, ist ein endogener Faktor mit zellprotektiver Wirksamkeit, insbesondere unter zellschädigenden Bedingungen mit begrenzter Sauerstoff- und Substratversorgung, wie z.B. bei Ischämie in verschiedensten Organen (z.B. Herz und Gehirn).

Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Die Wirkungen dieser Adenosin-Rezeptoren werden intrazellulär durch den Botenstoff cAMP vermittelt Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Adenosinrezeptor-selektive Liganden lassen sich nach ihrer Rezeptorselektivität in verschiedene Klassen einteilen, so z.B. in Liganden, die selektiv an die A1- oder die A2-Rezeptoren des Adenosin binden, bei letzteren auch beispielsweise solche, die selektiv an die A2a- oder die A2b-Rezeptoren des Adenosin binden. Auch sind Adenosinrezeptor-Liganden möglich, die selektiv an mehrere Subtypen der Adenosin-rezeptoren binden, so z.B. Liganden, die selektiv an die A1- und an die A2-, jedoch nicht an die A3-Rezeptoren des Adenosin binden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M.E. Olah, H. Ren, J. Ostrowski, K.A. Jacobson, G.L. Stiles, "Cloning, expression, and characterization of the unique bovine A1 adenosine receptor. Studies on the ligand binding site by site-directed mutagenesis." in J. Biol. Chem. 267 (1992) Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K.N. Klotz, J. Hessling, J. Hegler, C. Owman, B. Kull, B.B. Fredholm, M.J. Lohse, "Comparative pharmacology of human adenosine receptor subtypes - characterization of stably transfected receptors in CHO cells" in Naunyn Schmiedebergs Arch. Pharmacol. 357 (1998) Seiten 1-9, deren Offenbarung hiermit im vollen Umfang durch Bezugnahme eingeschlossen ist).

Bei den aus dem Stand der Technik bekannten, als "adenosinrezeptor-spezifisch" geltenden Liganden handelt es sich überwiegend um Derivate auf Basis des natürlichen Adenosins (S.-A. Poulsen und R.J. Quinn, "Adenosine receptors: new opportunities for future drugs" in Bioorganic and Medicinal Chemistry 6 (1998) Seiten 619-641; K. J. Broadley, "Drugs modulating adenosine receptors as potential therapeutic agents for cardiovascular diseases" in Exp. Opin. Ther. Patents 10 (2000) Seiten 1669-1692). Die aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Daher werden sie aufgrund der zuvor genannten Nachteile überwiegend nur für experimentelle Zwecke verwendet.

Aufgabe der vorliegenden Erfindung ist es, pharmakologisch aktive Substanzen aufzufinden oder bereitzustellen, die für die Prophylaxe und/oder Behandlung verschiedenster Erkrankungen, insbesondere Erkrankungen des Herz-Kreislauf-Systems (kardiovaskuläre Erkrankungen), geeignet sind und dabei vorzugsweise als Adenosinrezeptor-selektive Liganden wirken.

KANDEEL ET AL (HETEROCYCLIC COMMUNICATIONS, Bd. 3, Nr. 4, 1997, Seiten 371-380), WO0206237, WO0162233 und WO0125210 offenbaren strukturell verwandte Verbindungen der gleichen Wirkungsrichtung.

Die vorliegende Erfindung betrifft Verbindungen der Formel (I), 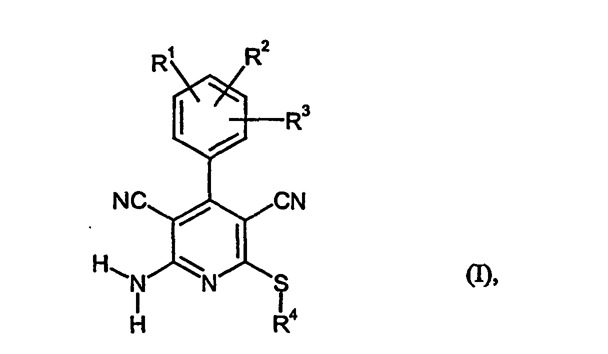 worin
- R¹ und R²: an benachbarte Phenylringatome gebunden sind und für eine Gruppe 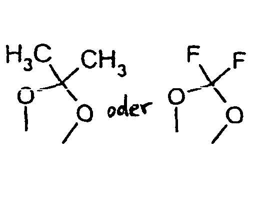 stehen,
- R³: Wasserstoff bedeutet,
und
- R⁴: Propenyl, Methyl, Ethyl oder n-Propyl bedeutet, wobei die Alkylreste ihrerseits bis zu zweifach, unabhängig voneinander, durch Hydroxy, Methoxy, Trifluormethyl, Trifluormethylthio, Fluor, Imidazolyl, gegebenenfalls durch Methyl substituiertes Thiazolyl, Pyridyl, Phenyl, das seinerseits wiederum durch Fluor, Cyano, Nitro, Methoxy, Methoxycarbonyl (-C(O)-O-CH₃) oder Methoxycarbonylmethyl (-CH₂-C(O)-O-CH₃) substituiert sein kann, Methoxycarbonyl (-C(O)-O-CH₃), Amido (-C(O)-NH₂) oder N-Methylamido (-C(O)-NH-CH₃) substituiert sein können,
und ihre Salze, Hydrate, Hydrate der Salze und Solvate.

2. Verbindungen nach Anspruch 1,
worin
- R¹ und R²: an benachbarte Phenylringatome gebunden sind und für eine Gruppe 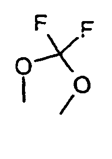
und ihre Salze, Hydrate, Hydrate der Salze und Solvate.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die Tautomeren der Verbindungen der Formel (I).

Salze der Verbindungen der Formel (I) können physiologisch unbedenkliche Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Im Rahmen der vorliegenden Erfindung haben die Substituenten, soweit nicht anders angegeben, die folgende Bedeutung:

Halogen steht im Allgemeinen für Fluor, Chlor, Brom oder Iod. Bevorzugt sind Fluor, Chlor oder Brom. Ganz besonders bevorzugt sind Fluor oder Chlor.

(C₁-C₈)-Alkyl, (C₁-C₆)-Alkyl bzw. (C₁-C₄)-Alkyl steht im Allgemeinen für einen geradkettigen oder verzweigten Alkylrest mit 1 bis 8, 1 bis 6 bzw. 1 bis 4 Kohlenstoffatomen. Bevorzugt ist ein geradkettiger oder verzweigter Alkylrest mit 1 bis 6 Kohlenstoffatomen. Besonders bevorzugt ist ein geradkettiger oder verzweigter Alkylrest mit 1 bis 4 Kohlenstoffatomen. Beispielsweise seien genannt: Methyl, Ethyl, n-Propyl, Isopropyl, n-Butyl, sec-Butyl, Isobutyl und tert.-Butyl.

(C₂-C₄)-Alkenyl stehen im Allgemeinen für einen geradkettigen oder verzweigten Alkenylrest mit 2 bis 4 Kohlenstoffatomen. Beispielsweise seien genannt: Vinyl, Allyl, Isopropenyl und n-But-2-en-1-yl.

(C₂-C₄)-Alkinyl steht im Allgemeinen für einen geradkettigen oder verzweigten Alkinylrest mit 2 bis 4 Kohlenstoffatomen. Beispielsweise seien genannt: Ethinyl, n-Prop-2-in-1-yl und n-But-2-in-1-yl.

(C₁-C₈)-Alkoxy, (C₁-C₆)-Alkoxy bzw. (C₁-C₄)-Alkoxy steht im Allgemeinen für einen geradkettigen oder verzweigten Alkoxyrest mit 1 bis 8, 1 bis 6 bzw. 1 bis 4 Kohlenstoffatomen. Bevorzugt ist ein geradkettiger oder verzweigter Alkoxyrest mit 1 bis 6 Kohlenstoffatomen. Besonders bevorzugt ist ein geradkettiger oder verzweigter Alkoxyrest mit 1 bis 4 Kohlenstoffatomen. Beispielsweise seien genannt: Methoxy, Ethoxy, n-Propoxy, Isopropoxy, n-Butoxy, Isobutoxy, sec-Butoxy, tert.-Butoxy.

(C₁-C₄)-Alkoxycarbonyl steht im Allgemeinen für einen geradkettigen oder verzweigten Alkoxyrest mit 1 bis 4 Kohlenstoffatomen, der über eine Carbonylgruppe verknüpft ist. Beispielsweise seien genannt: Methoxycarbonyl, Ethoxycarbonyl, n-Propoxycarbonyl, Isopropoxycarbonyl und t-Butoxycarbonyl.

Mono- oder Di-(C₁-C₄)-alkylamino steht im Rahmen der Erfindung für eine AminoGruppe mit einem oder mit zwei gleichen oder verschiedenen geradkettigen oder verzweigten Alkylsubstituenten, die jeweils 1 bis 4 Kohlenstoffatome aufweisen. Beispielsweise seien genannt: Methylamino, Ethylamino, n-Propylamino, Isopropylamino, t-Butylamino, *N,N-*Dimethylamino, *N,N*-Diethylamino, *N*-Ethyl-*N*-methylamino, *N*-Methyl-*N*-n-propylamino, *N*-Isopropyl-*N*-n-propylamino und *N*-t-Butyl-*N*-methylamino.

(C₃-C₇)-Cycloalkyl bzw. (C₃-C₆)-Cycloalkyl steht im Allgemeinen für einen cyclischen Alkylrest mit 3 bis 7 bzw. 3 bis 6 Kohlenstoffatomen. Bevorzugt sind cyclische Alkylreste mit 3 bis 6 Kohlenstoffatomen. Beispielsweise seien genannt: Cyclopropyl, Cyclobutyl, Cyclopentyl oder Cyclohexyl.

(C₆-C₁₀)-Aryl steht im Allgemeinen für einen aromatischen Rest mit 6 bis 10 Kohlenstoffatomen. Bevorzugte Arylreste sind Phenyl und NaphthyL

(C₆-C₁₀)-Aryloxy steht im Allgemeinen für einen wie zuvor definierten aromatischen Rest, der über ein Sauerstoffatom verknüpft ist.

5- bis 10-gliedriges Heteroaryl mit bis zu 3 Heteroatomen und/oder Hetero-Kettenglieder aus der Reihe N, NO (N-Oxid), O und/oder S steht im Allgemeinen für einen mono- oder bicyclischen, gegebenenfalls benzokondensierten Heteroaromaten, der über ein Ringkohlenstoffatom des Heteroaromaten, gegebenenfalls auch über ein Ringstickstoffatom des Heteroaromaten, verknüpft ist. Beispielsweise seien genannt: Pyridyl, Pyridyl-N-oxid, Pyrimidyl, Pyridazinyl, Pyrazinyl, Thienyl, Furyl, Pyrrolyl, Pyrazolyl, Imidazolyl, Triazolyl, Thiazolyl, Oxazolyl, Oxdiazolyl, Isoxazolyl, Benzofuranyl, Benzothienyl oder Benzimidazolyl. Aus dieser Definition leiten sich analog die entsprechenden Heteroaromaten mit weniger Heteroatomen wie z.B. mit einem oder 2 Heteroatomen aus der Reihe N, O und/oder S oder geringerer Ringgröße wie z.B. 5- oder 6-gliedriges Heteroaryl ab. Im Allgemeinen gilt, dass 5- oder 6-gliedrige aromatische Heterocyclen mit einem oder 2 Heteroatomen aus der Reihe N, O und/oder S bevorzugt sind. Beispielsweise seien genannt: Pyridyl, Pyrimidyl, Pyridazinyl, Furyl, Imidazolyl oder Thienyl.

5- bis 7-gliedriger Heterocyclus steht im Allgemeinen für einen gesättigten oder teilweise ungesättigten, gegebenenfalls benzokondensierten Heterocyclus mit bis zu 3 Heteroatomen aus der Reihe N, O und/oder S. Beispielsweise seien genannt: Tetrahydrofuryl, Pyrrolidinyl, Pyrrolinyl, Dihydropyridinyl, Piperidinyl, Piperazinyl, Morpholinyl, Thiomorpholinyl, Hexahydropyranyl. Aus dieser Definition leiten sich analog die entsprechenden Heterocyclen mit weniger Heteroatomen wie z.B. mit einem oder 2 Heteroatomen aus der Reihe N, O und/oder S oder geringerer Ringgröße wie z.B. 5- oder 6-gliedriges Heterocyclyl ab.Bevorzugt sind gesättigte Heterocyclen mit bis zu 2 Heteroatomen aus der Reihe N, O und/oder S, insbesondere Piperidinyl, Piperazinyl, Morpholinyl und Pyrrolidinyl.

Besonders bevorzugt sind Verbindungen der Formel (I),
worin
- R¹ und R²: an benachbarte Phenylringatome gebunden sind und für eine Gruppe 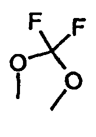 oder 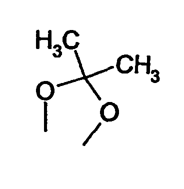 stehen,
- R³: Wasserstoff bedeutet,
und
- R⁴: Methyl, Ethyl oder n-Propyl bedeutet, wobei die Alkylreste ihrerseits bis zu zweifach, unabhängig voneinander, durch Hydroxy, Trifluormethyl, Trifluormethylthio, Fluor, Imidazolyl, gegebenenfalls durch Methyl substituiertes Thiazolyl, Phenyl, das seinerseits wiederum durch Cyano, Nitro, Methoxycarbonyl (-C(O)-O-CH₃) oder Methoxycarbonylmethyl (-CH₂-C(O)-O-CH₃) substituiert ist, oder Amido (-C(O)-NH₂) substituiert sein können,
und ihre Salze, Hydrate, Hydrate der Salze und Solvate.

Ebenfalls besonders bevorzugt sind die Verbindungen der Beispiele 3, 42, 43, 44, 45, 47, 52, 53, 54 und ihre Salze, Hydrate, Hydrate der Salze und Solvate.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen, beliebig kombiniert werden. Sie gelten für die Endprodukte sowie für die Vor- und Zwischenprodukte entsprechend.

Die vorliegende Erfindung betrifft weiterhin ein Verfahren zur Herstellung der Verbindungen der Formel (I), dadurch gekennzeichnet, dass man

Verbindungen der Formel (II) 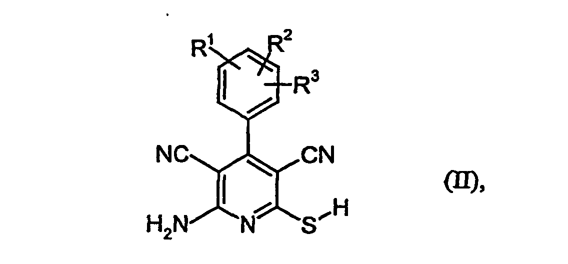 in welcher
die Reste R¹, R² und R³ die oben angegebene Bedeutung haben,
in einem Lösemittel, gegebenenfalls in Anwesenheit einer Base, mit Verbindungen der Formel (III)

R⁴-X (III),

in welcher
- R⁴: die oben angegebene Bedeutung hat und
- X: für eine Abgangsgruppe wie beispielsweise Halogen, insbesondere Chlor, Brom oder Iod, oder Mesylat, Tosylat, Triflat oder 1-Imidazolyl steht,
umsetzt.

Das zuvor beschriebene Verfahren kann durch folgendes Formelschema beispielhaft erläutert werden:

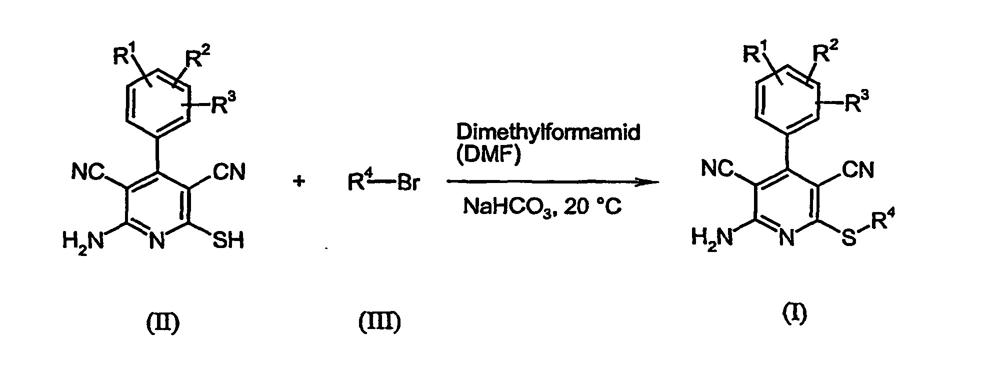

Als Lösemittel für das erfindungsgemäße Verfahren eignen sich alle organischen Lösemittel, die unter den Reaktionsbedingungen inert sind. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, Dimethylformamid, Acetonitril, Pyridin, Dimethylsulfoxid (DMSO), chlorierte Kohlenwasserstoffe wie Dichlormethan, Chlorbenzol oder Dichlorethan. Wasser ist als Lösemittel ebenso geeignet. Bevorzugt ist Dimethylformamid. Ebenso ist es möglich, Gemische der zuvor genannten Lösemittel einzusetzen.

Als Basen eignen sich die üblichen anorganischen oder organischen Basen. Hierzu gehören bevorzugt Alkalihydroxide wie beispielsweise Natrium- oder Kaliumhydroxid oder Alkalicarbonate wie Natrium- oder Kaliumcarbonat oder Natrium- oder Kaliumhydrogencarbonat oder Natrium- oder Kaliummethanolat oder Natrium- oder Kaliumethanolat oder Kalium-tert.-butylat oder aber Amide wie Natriumamid, Lithium-bis-(trimethylsilyl)amid oder Lithiumdiisopropylamid oder metallorganische Verbindungen wie Butyllithium oder Phenyllithium oder aber auch Amine wie Triethylamin und Pyridin. Bevorzugt sind die Alkalicarbonate oder -hydrogencarbonate, insbesondere Natriumcarbonat oder Natriumhydrogencarbonat.

Die Base kann hierbei in einer Menge von 1 bis 10 Mol, bevorzugt von 1 bis 5 Mol, insbesondere 1 bis 4 Mol, bezogen auf 1 Mol der Verbindungen der Formel (II) eingesetzt werden.

Die Reaktion erfolgt im Allgemeinen in einem Temperaturbereich von -78°C bis +120°C, bevorzugt im Bereich von -78°C bis +40°C, insbesondere bei Raumtemperatur.

Die Umsetzung kann bei normalem, erhöhtem oder erniedrigtem Druck durchgeführt werden (z.B. im Bereich von 0,5 bis 5 bar). Im Allgemeinen arbeitet man bei Normaldruck.

Verbindungen der Formel (III) sind kommerziell erhältlich, dem Fachmann bekannt oder nach literaturüblichen Methoden herstellbar.

Verbindungen der Formel (II) sind dem Fachmann bekannt oder nach literaturüblichen Methoden herstellbar. Insbesondere kann auf die folgenden Druckschriften verwiesen werden, deren jeweiliger Inhalt durch Bezugnahme eingeschlossen wird:
- Dyachenko et al., Russian Journal of Chemistry, Vol. 33, No. 7, 1997, Seiten 1014-1017 und Vol. 34, No. 4, 1998, Seiten 557-563;
- Dyachenko et al., Chemistry of Heterocyclic Compounds, Vol. 34, No. 2, 1998, Seiten 188-194;
- Qintela et al., European Journal of Medicinal Chemistry, Vol. 33, 1998, Seiten 887-897;
- Kandeel et al., Zeitschrift für Nahuforschung 42b, 107-111 (1987).

Verbindungen der Formel (II) können darüber hinaus beispielsweise auch aus Verbindungen der Formel (IV) durch Umsetzung mit einem Alkalisulfid hergestellt werden.

Diese Herstellungsmethode kann durch folgendes Formelschema beispielhaft erläutert werden:

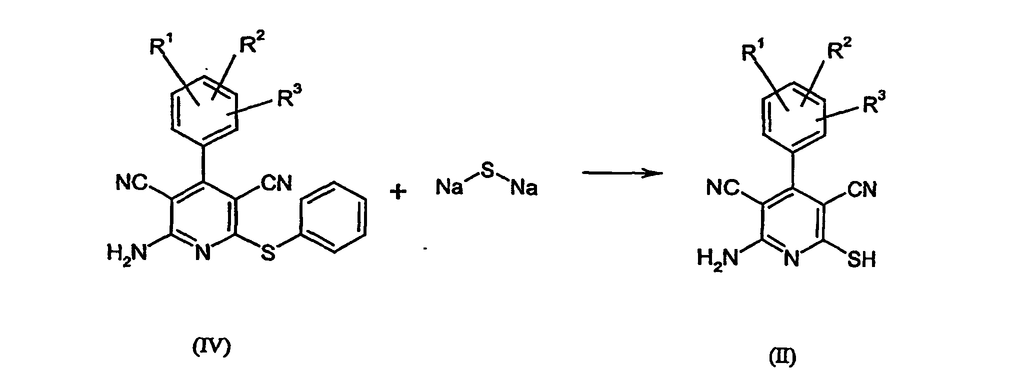

Als Alkalisulfid wird vorzugsweise Natriumsulfid in einer Menge von 1 bis 10 Mol, bevorzugt 1 bis 5 Mol, insbesondere 1 bis 4 Mol, bezogen auf 1 Mol der Verbindungen der Formel (IV) eingesetzt.

Als Lösungsmittel geeignet sind alle organischen Lösungsmittel, die unter den Reaktionsbedingungen inert sind. Hierzu gehören N,N-Dimethylformamid, N-Methylpyrrolidinon, Pyridin und Acetonitril. Bevorzugt ist N,N-Dimethylformamid. Ebenso ist es möglich, Gemische der zuvor genannten Lösungsmittel einzusetzen.

Die Reaktion erfolgt im Allgemeinen in einem Temperaturbereich von +20°C bis +150°C, bevorzugt im Bereich von +20°C bis +120°C, insbesondere bei +60°C bis +100°C.

Die Umsetzung kann bei normalem, erhöhtem oder erniedrigtem Druck durchgeführt werden (z.B. im Bereich von 0,5 bis 5 bar). Im Allgemeinen arbeitet man bei Normaldruck.

Verbindungen der Formel (IV) sind dem Fachmann bekannt oder nach üblichen, literaturbekannten Methoden herstellbar. Insbesondere kann auf die Druckschrift Kambe et aL, Synthesis, 531 (1981) verwiesen werden, deren Inhalt durch Bezugnahme eingeschlossen wird.

Überraschenderweise zeigen die Verbindungen der Formel (I) ein nicht vorhersehbares pharmakologisches Wirkspektrum und sind daher insbesondere zur Prophylaxe und/oder Behandlung von Erkrankungen geeignet.

Die Verbindungen der Formel (I) sind zur Prophylaxe und/oder Behandlung einer ganzen Reihe von Erkrankungen geeignet, so beispielsweise insbesondere von Erkrankungen des Herzkreislaufsystems (kardiovaskulären Erkrankungen).

Im Sinne der vorliegenden Erfindung sind unter Erkrankungen des Herz-Kreislauf-Systems bzw. kardiovaskulären Erkrankungen beispielsweise insbesondere die folgenden Erkrankungen zu verstehen: Koronare Herzkrankheit, Hypertonie (Bluthochdruck), Restenose wie z.B. Restenose nach Ballondilatation von peripheren Blutgefäßen, Arteriosklerose, Tachykardien, Arrhytbmien, periphere und kardiale Gefäßerkrankungen, stabile und instabile Angina pectoris und Vorhofflimmern.

Weiterhin eignen sich die Verbindungen der Formel (I) beispielsweise insbesondere auch zur Reduktion des von einem Infarkt betroffenen Myokardbereichs.

Des weiteren eignen sich die Verbindungen der Formel (I) beispielsweise insbesondere zur Prophylaxe und/oder Behandlung von thromboembolischen Erkrankungen und Ischämien wie Myokardinfarkt, Hirnschlag und transitorischen ischämischen Attacken.

Weitere Indikationsgebiete, für das sich die Verbindungen der Formel (I) eignen, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandlung von inflammatorischen Erkrankungen, wie z.B. Asthma und entzündlichen Dermatosen, von neuroinflammatorischen Erkrankungen des Zentralnervensystems, wie beispielsweise Zustände nach Hirninfarkt, der Alzheimer-Erkrankung, weiterhin auch von neurodegenerative Erkrankungen wie der Parkinson-Erkrankung, sowie von Schmerzzuständen und Krebs.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasen, zystische Fibrose (Mukoviszidose) und pulmonale Hypertonie.

Des weiteren kommen die Verbindungen der Formel (I) auch beispielsweise insbesondere für die Prophylaxe und/oder Behandlung von Leberfibrose und Leberzirrhose in Betracht.

Schließlich kommen die Verbindungen der Formel (I) beispielsweise insbesondere auch für die Prophylaxe und/oder Behandlung von Diabetes, insbesondere Diabetes mellitus, in Betracht.

Die vorliegende Erfindung betrifft auch die Verwendung der Substanzen der Formel (I) zur Herstellung von Arzneimitteln und pharmazeutischen Zusammensetzungen zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Substanzen der Formel (I).

Die pharmazeutische Wirksamkeit der zuvor genannten Verbindungen der Formel (I) lässt sich durch ihre Wirkung als selektive Liganden an einzelnen oder mehreren Subtypen der Adenosin-Rezeptoren, insbesondere als selektive Liganden an Adenosin-A1-, Adenosin-A2a- und/oder Adenosin-A2b-Rezeptoren, vorzugsweise als selektive Liganden an Adenosin-A1- und/oder Adenosin-A2b-Rezeptoren erklären.

Als "selektiv" werden im Rahmen der vorliegenden Erfindung solche Adenosin-rezeptor-Liganden bezeichnet, bei denen einerseits eine deutliche Wirkung an einem oder mehreren Adenosin-Rezeptor-Subtypen und andererseits keine oder eine deutlich schwächere Wirkung an einem oder mehreren anderen Adenosin-Rezeptor-Subtypen zu beobachten ist, wobei bezüglich der Testmethoden für die Wirk-Selektivität Bezug genommen wird auf die im Abschnitt A. II. beschriebenen Testmethoden.

Ein Vorteil der erfindungsgemäßen Verbindungen der Formel (I) ist, dass sie gegenüber Adenosinrezeptor-Liganden des Standes der Technik selektiver wirken.

Insbesondere wirken Verbindungen der Formel (I), worin R¹ und R² für eine Gruppe -O-CH₂-O-, -O-CH₂-CH₂-O- oder -O-CH(CH₂OH)-CH₂-O- stehen, im Allgemeinen agonistisch an Adenosin-A1-Rezeptoren.

Insbesondere wirken Verbindungen der Formel (I), worin R¹ und R² für eine Gruppe -O-CF₂-O- stehen, im Allgemeinen antagonistisch an Adenosin-A1-Rezeptoren.

Die Rezeptorselektivität kann bestimmt werden durch die biochemische Messung des intrazellulären Botenstoffes cAMP in den transfizierten Zellen, die spezifisch nur einen Subtyp der Adenosinrezeptoren exprimieren. Im Falle von A2a- bzw. A2b-Agonisten (Kopplung bevorzugt über Gs-Proteine) wird dabei ein Anstieg des intrazellulären cAMP-Gehaltes, im Falle von A2a- bzw.A2b-Antagonisten eine Abnahme des intrazellulären cAMP-Gehaltes nach Vorstimulation mit Adenosin oder Adenosin ähnlichen Substanzen beobachtet (siehe Druckschriften B. Kull, G. Arslan, C. Nilsson, C. Owman, A. Lorenzen, U. Schwabe, B.B. Fredholm, "Differences in the order of potency for agonists but not antagonists at human and rat adenosine A2A receptors", Biochem. Pharmacol., 57 (1999) Seiten 65-75; und S.P. Alexander, J. Cooper, J. Shine, S.J. Hill, "Characterization of the human brain putative A2B adenosine receptor expressed in Chinese hamster ovary (CHO.A2B4) cells", Br. J. Pharmacol., 119 (1996) Seiten 1286-90, deren jeweilige Offenbarung hiermit durch Bezugnahme eingeschlossen ist). Entsprechend führen A1-Agonisten (Kopplung bevorzugt über Gi-Proteine) zu einer Abnahme und A1-Antagonisten zu einem Anstieg im cAMP-Gehalt.

So eignen sich Verbindungen der Formel (I), die selektiv an Adenosin-A1-Rezeptoren binden, bevorzugt zur Myokard-Protektion und zur Prophylaxe und/oder Behandlung von Tachykardien, Vorhof-Arrhythmien, Herzinsuffizienz, Herzinfarkt, akutem Nierenversagen, Diabetes, sowie von Schmerzzuständen.

Verbindungen der Formel (I), die selektiv an Adenosin-A2a-Rezeptoren binden, sind bevorzugt zur Prophylaxe und/oder Behandlung von thrombo-embolischen Erkrankungen, von neurodegenerativen Erkrankungen wie Morbus Parkinson sowie zur Wundheilung geeignet.

Verbindungen der Formel (I), die selektiv an Adenosin-A2b-Rezeptoren binden, eignen sich bevorzugt zur Prophylaxe und/oder Therapie der Leberfibrose, des Herzinfarkts, von neuroinflammatorischen Erkrankungen, der Alzheimer-Erkrankung, von urogenitaler Inkontinenz sowie von Atemwegserkrankungen wie beispielsweise Asthma und chronischer Bronchitis.

Weiterer Gegenstand der vorliegenden Erfindung sind Arzneimittel und pharmazeutische Zusammensetzungen, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der Formel (I) kommen alle üblichen Applikationsformen in Betracht, d.h. also oral, parenteral, inhalativ, nasal, sublingual, rektal, lokal, wie z.B. bei Templaten oder Stents, oder äußerlich wie z.B. transdermal. Bei der parenteralen Applikation sind insbesondere intravenöse, intramuskuläre, subkutane Applikation zu nennen, z.B. als subkutanes Depot. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird Im Allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten auch allgemein übliche Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserern oder Farbstoffen versetzt werden.

Im Allgemeinen hat es sich als vorteilhaft erwiesen, bei parenteraler Applikation Mengen von etwa 0,1 bis etwa 10 000 µg/kg, vorzugsweise etwa 1 bis etwa 1 000 µg/kg, insbesondere etwa 1 µg/kg bis etwa 100 µg/kg Körpergewicht, zur Erzielung wirksamer Ergebnisse zu verabreichen. Bei oraler Applikation beträgt die Menge etwa 0,1 bis etwa 10 mg/kg, vorzugsweise etwa 0,5 bis etwa 5 mg/kg, insbesondere etwa 1 bis etwa 4 mg/kg Körpergewicht.

In Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt, kann es gegebenenfalls erforderlich sein, von den genannten Mengen abzuweichen.

Die vorliegende Erfindung wird an den folgenden Beispielen veranschaulicht, die die Erfindung jedoch keinesfalls beschränken.

### A. Bewertung der physiologischen Wirksamkeit

### I. Nachweis der kardiovaskulären Wirkung

### Langendorff-Herz der Ratte:

Narkotisierten Ratten wird nach Eröffnung des Brustkorbes das Herz entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

### II. Nachweis der Rezeptorselektivität

### a) Adenosin-A1-, A2a-, A2b- und A3-Rezeptorselektivität

Zellen der permanenten Linie CHO (Chinese Hamster Ovary) werden stabil mit der cDNA für die Adenosin-Rezeptor-Subtypen A1, A2a, A2b und A3 transfiziert. Die Bindung der Substanzen an die A2a- oder A2b-Rezeptorsubtypen wird bestimmt durch Messung des inträzellulären cAMP-Gehaltes in diesen Zellen mit einem konventionellen radioimmunologischen Assay (cAMP-RIA).

Im Falle der Wirkung der Substanzen als Agonisten kommt es als Ausdruck der Bindung der Substanzen zu einem Anstieg des intrazellulären cAMP-Gehaltes. Als Referenzverbindung dient in diesen Experimenten die Adenosin-analoge Verbindung NECA (5-N-Ethylcarboxamido-adenosin), die nicht selektiv, aber mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

Die Adenosin-Rezeptoren A1 und A3 sind an ein Gi-Protein gekoppelt, d.h. eine Stimulation dieser Rezeptoren führt zu einer Inhibition der Adenylatcyclase und somit zu einer Senkung des intrazellulären cAMP-Spiegels. Zur Identifizierung von A1/A3-Rezeptor-Agonisten wird die Adenylatcyclase mit Forskolin stimuliert. Eine zusätzliche Stimulation der A1/A3-Rezeptoren hemmt jedoch die Adenylatcyclase, so dass A1/A3-Rezeptor-Agonisten über einen vergleichsweise geringen Gehalt der Zelle an cAMP detektiert werden können.

Für den Nachweis einer antagonistischen Wirkung an Adenosin-Rezeptoren werden die mit dem entsprechenden Rezeptor transfizierten, rekombinanten Zellen mit NECA vorstimuliert und die Wirkung der Substanzen auf eine Reduktion des intrazellulären cAMP-Gehalts durch diese Vorstimulation untersucht. Als Referenzverbindung dient in diesen Experimenten XAC (xanthine amine congener), die nicht selektiv, aber mit hoher Affinität an alle Adenosinrezeptor-Subtypen bindet und eine antagonistische Wirkung besitzt (Müller, C.E., Stein, B., Adenosine receptor antagonists: structures and potential therapeutic applications, Current Pharmaceutical Design, 2 (1996), 501-530).

### b) Adenosin-A1-, A2a-, A2b- Rezeptorselektivität

Zellen der permanenten Linie CHO (Chinese Hamster Ovary) werden stabil mit der cDNA für die Adenosin-Rezeptor-Subtypen A1, A2a, A2b transfiziert. Die Adenosin A1 Rezeptoren sind über Gᵢ-Proteine und die Adenosin A2a und A2b Rezeptoren über Gs-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luciferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanztest-Screening wird das folgende Testprotokoll verwendet:

Die Stammkulturen wird in DMEM/F12 Medium mit 10 % FCS (fötales Kälberserum) bei 37°C unter 5 % CO₂ gezüchtet und jeweils nach 2-3 Tagen 1:10 gesplittet. Testkulturen werden von 1000 bis 3000 Zellen pro Napf in 384-well Platten ausgesät und ca. 48 Stunden bei 37°C angezogen. Dann wird das Medium durch eine physiologische Kochsalzlösung (130 mM NaCl, 5 mM KCl, 2 mM CaCl₂, 20 mM HEPES, 1 mM MgCl₂•6H₂O, 5 mM NaHCO₃, pH 7,4) ersetzt. Die in DMSO gelösten Substanzen werden 3 mal 1:10 mit dieser physiologischen Kochsalzlösung verdünnt und zu den Testkulturen pipettiert (maximale DMSO-Endkonzentration im Testansatz: 0,5 %) So erhält man Substanzendkonzentrationen von beispielsweise 5 µM bis 5 nM. 10 Minuten später wird Forskolin zu den A1 Zellen zugegeben und anschließend werden alle Kulturen für 4 Stunden bei 37°C inkubiert. Danach wird zu den Testkulturen 35 µl Lösung, bestehend zu 50 % aus Lysereagenz (30 mM di-Natriumhydrogenphosphat, 10 % Glycerin, 3 % TritonX100, 25 mM TrisHCl, 2 mM Dithiotreitol (DTT), pH 7,8) und zu 50 % aus Luciferase Substrat Lösung (2,5 mM ATP, 0,5 mM Luciferin, 0,1 mM Coenzym A, 10 mM Tricin, 1,35 mM MgSO₄, 15 mM DTT, pH 7,8) zugegeben, ca. 1 Minute geschüttelt und die Luciferase-Aktivität mit einem Kamerasystem gemessen.

### B. Ausführungsbeispiele

### Verwendete Abkürzungen:

- DMSO: Dimethylsulfoxid
- d. Th.: der Theorie
- HPLC: Hochdruck-, Hochleistungsflüssigchromatographie
- NMR: Kernresonanzspektroskopie
- DMF: Dimethylformamid
- i. V.: im Vakuum

### Beispiel 1 (Referenzbeispiel)

### 2-Amino-4-(1,3-benzodioxol-5-yl)-6-(2-hydroxyethyl)sulfanyl-3,5-pyridin-dicarbonitril

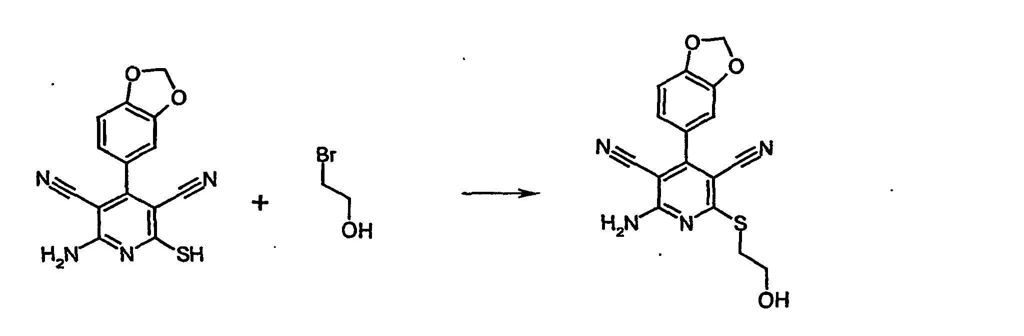

75 mg (0,19 mmol) 2-Amino-4-(1,3-benzodioxol-5-yl)-6-sulfanyl-3,5-pyridindicarbonitril [hergestellt analog zu Dyachenko et al., Russian Journal of Chemistry 33 (7), 1014-1017 (1997); 34 (4), 557-563 (1998)] werden in 1 ml DMF zusammen mit 47 mg (0,38 mmol) 2-Bromethanol und 63 mg (0,75 mmol) Natriumhydrogencarbonat über Nacht bei Raumtemperatur gerührt. Dann wird Wasser zugegeben und das ausgefallene Produkt abgesaugt und i.V. getrocknet.
Ausbeute: 55 mg (85,8 % d. Th.)
Massenspektrum: gesuchte Molmasse 340, gefunden [M+H]⁺ = 341
¹H-NMR-Spektrum [DMSO-d₆]: δ = 3,4 [2H] tr; 3,65 [2H] q; 5,0 [1H] tr; 6,15 [2H] s; 7,0 - 7,2 [3H] m; 7,8 - 8,2 [2H] s breit.

### Beispiel 2 (Referenzbeispiel)

### 2-Amino-4-(1,3-benzodioxol-5-yl)-6-(benzylsulfanyl)-3,5-pyridindicarbonitril

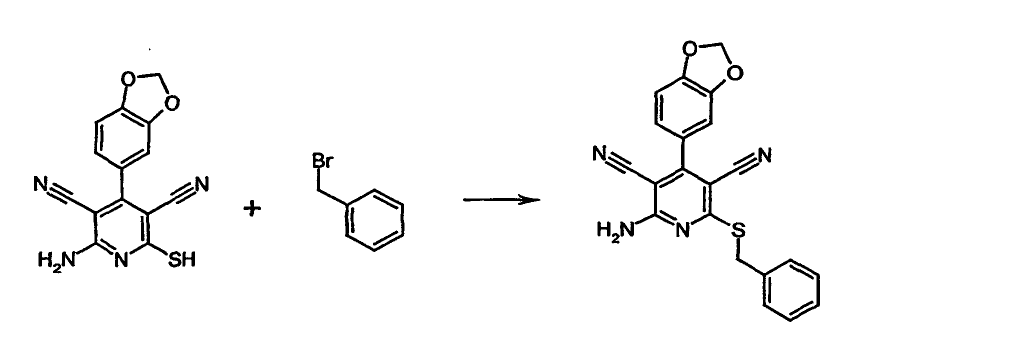

Die Umsetzung wurde analog zu Beispiel 1 durchgeführt.
Ausbeute: 74 mg (100 % d. Th.)
Massenspektrum: gesuchte Molmasse 386, gefunden [M+H]⁺ = 387
¹H-NMR-Spektrum [DMSO-d₆]: δ = 4,5 [2H] s; 6,15 [2H] s; 7,0 - 7,2 [3H] m; 7,3 - 7,6 [5H] m; 7,8 - 8,2 [2H] s breit.

### Beispiel 3

### 2-Amino-4-(2,2-difluoro-1,3-benzodioxol-5-yl)-6-[(2-pyridinylmethyl)sulfanyl]-3,5-pyridindicarbonitril

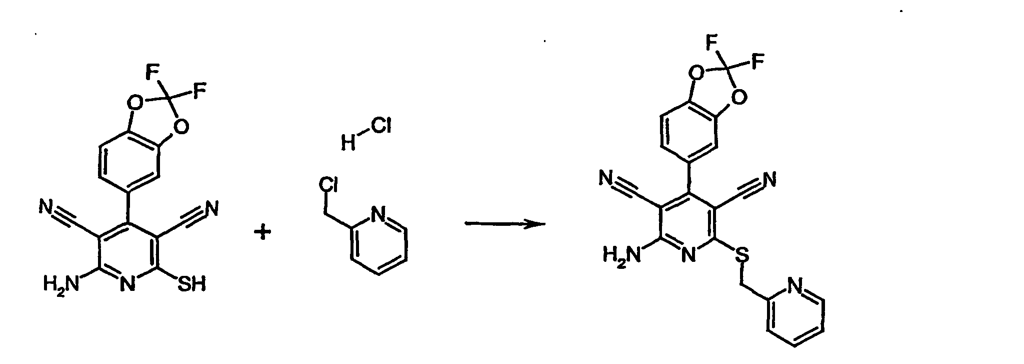

Die Umsetzung wurde analog zu Beispiel 1 durchgeführt.
Ausbeute: 50 mg (79 % d. Th.)
Massenspektrum: gesuchte Molmasse 423, gefunden [M+H]⁺ = 424
¹H-NMR-Spektrum [DMSO-d₆]: δ = 4,6 [2H] s; 6,8 [1H] m; 6,95 [1H] dd; 7,6 - 7,8 [4H] m; 7,9 - 8,4 [2H] s breit; 8,55 [1H] d.

### Beispiel 4 (Referenzbeispiel)

### 2-Amino-6-(benzylsulfanyl)-4-(2,3-dihydro-1,4-benzodioxin-6-yl)-3,5-pyridindicarbonitril

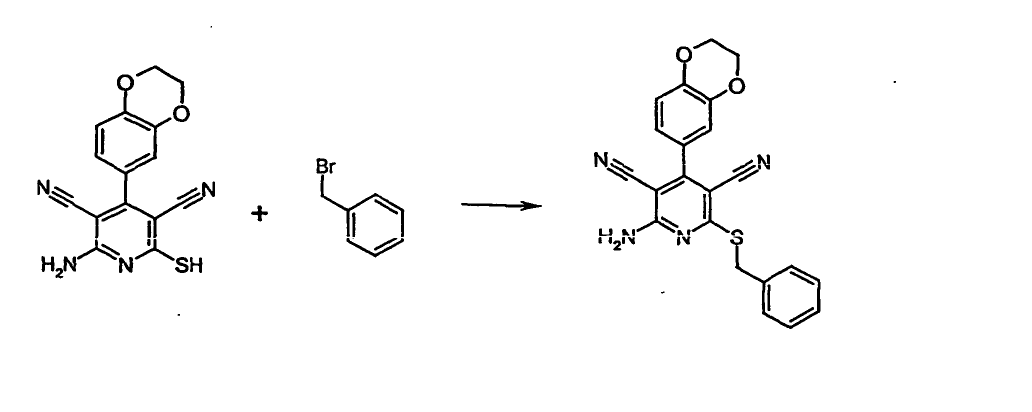

100 mg (0,32 mmol) 2-Amino-6-sulfanyl-4-(2,3-dihydro-1,4-benzodioxin-6-yl)-3,5-pyridindicarbonitril [hergestellt analog zu Dyachenko et al., Russian Journal of Chemistry 33 (7), 1014-1017 (1997); 34 (4), 557-563 (1998)] werden in 2 ml DMF zusammen mit 110 mg (0,64 mmol) Benzylbromid und 108 mg (1,29 mmol) Natriumhydrogencarbonat 5,5 h bei Raumtemperatur gerührt. Dann wird Wasser zugegeben und dreimal mit Ethylacetat extrahiert. Die vereinigten organischen Phasen werden mit Magnesiumsulfat getrocknet und i.V. eingedampft. Der Rückstand wird in Diethylether aufgenommen und ergibt nach erneutem Eindampfen kristallines Produkt.
Ausbeute: 106 mg (82 % d. Th.)
Massenspektrum: gesuchte Molmasse 400, gefunden [M+H]⁺ = 401
¹H-NMR-Spektrum [DMSO-d₆]: δ = 4,3 [4H] m; 4,5 [2H] s; 6,9 - 7,1 [3H] m; 7,2 - 7, 4 [3H] m; 7,5 [2H] m; 7,8 - 8,2 [2H] s breit.

### Beispiel 5 (Referenzbeispiel)

### 2-Amino-6-((2-hydroxyethyl)sulfanyl)-4-(2,3-dihydro-1,4-benzodioxin-6-yl)-3,5-pyridindicarbonitril

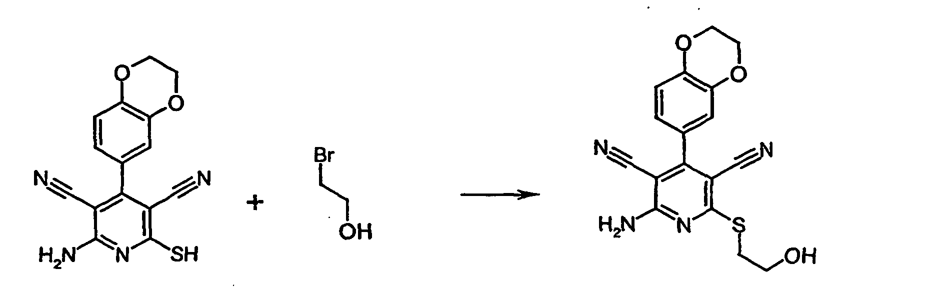

Die Umsetzung wurde analog zu Beispiel 1 durchgeführt.
Ausbeute: 15 mg (13 % d. Th.)
Massenspektrum: gesuchte Molmasse 354, gefunden [M+H]⁺ = 355
¹H-NMR-Spektrum [DMSO-d₆]: δ = 3,4 [2H] tr; 3,65 [2H] q; 4,3 [4H] s; 5,0 [1H] tr; 7,0 - 7,1 [3H] m; 7,8 - 8,1 [2H] s breit.

### Beispiel 6 (Referenzbeispiel)

### 2-Amino-6-[(2-hydroxyethyl)sulfanyl)-4-[2-(hydroxymethyl)-2,3-dihydro-1,4-benzodioxin-6-yl]-3,5-pyridindicarbonitril

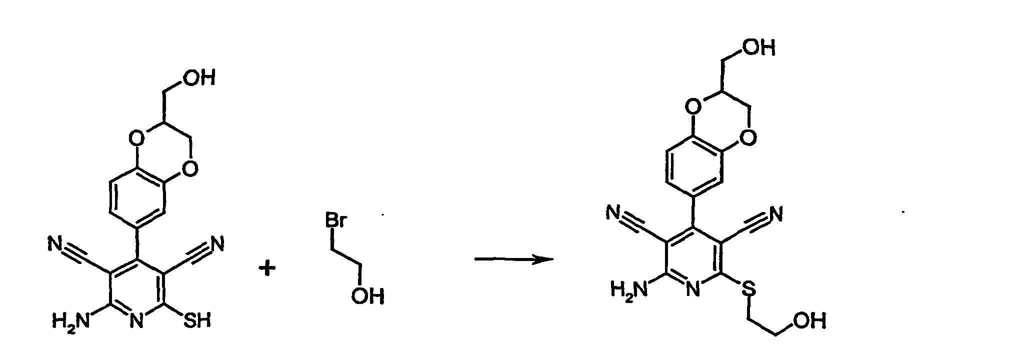

30 mg (0,09 mmol) 2-Amino-6-sulfanyl-4-[2-(hydroacymethyl)-2,3-dihydro-1,4-benzodioxin-6-yl]-3,5-pyridindicarbonitril [hergestellt analog zu Dyachenko et al., Russian Journal of Chemistry 33 (7), 1014-1017 (1997); 34 (4), 557-563 (1998)] werden in 1,5 ml DMF zusammen mit 22 mg (0,18 mmol) 2-Hydroxyethylbromid und 29 mg (0,35 mmol) Natriumhydrogencarbonat über Nacht bei Raumtemperatur gerührt. Die Reaktionslösung wird direkt durch präparative HPLC an Reversed Phase-Kieselgel gereinigt.
Ausbeute: 2,1 mg (6 % d. Th.)
Massenspektrum: gesuchte Molmasse 384, gefunden [M+H]⁺ = 385
¹H-NMR-Spektrum [DMSO-d₆]: δ = 3,3 [2H] tr; 3,65 [4H] m; 4,05 [1H] dd; 4,3 [1H] m; 4,4 [1H] dd; 5,0 [1H] tr; 5,15 [1H] trm; 7,0 - 7,1 [3H] m; 7,8 - 8,1 [2H] s breit.

### Beispiel 7 (Referenzbeispiel)

### 2-Amino-6-[benzylsulfanyl]-4-[2-(hydroxymethyl)-2,3-dihydro-1,4-benzodioxin-6-yl]-3,5-pyridindicarbonitril

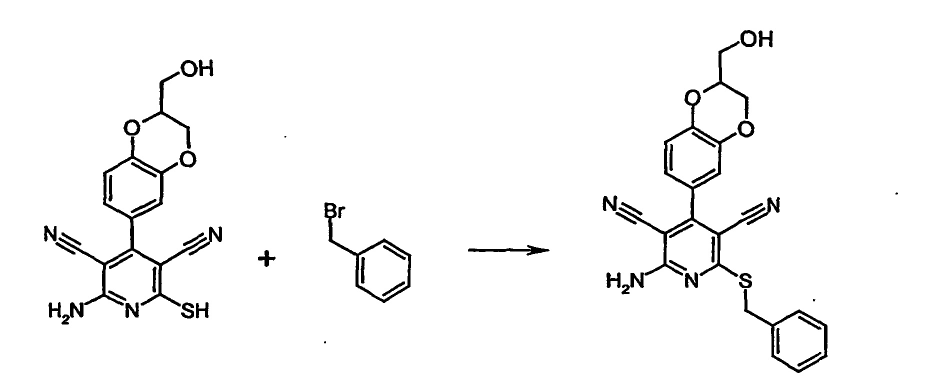

Die Umsetzung wurde analog zu Beispiel 6 durchgeführt.
Ausbeute: 4,6 mg (12 % d. Th.)
Massenspektrum: gesuchte Molmasse 430, gefunden [M+H]⁺ = 431
¹H-NMR-Spektrum [DMSO-d₆]: δ = 3,7 [2H] m; 4,05 [1H] dd; 4,3 [1H] m; 4,4 [1H] dd; 4,5 [2H] s; 5,1 [1H] tr; 7,0 - 7,1 [3H] m; 7,2-7,6 [5H] m; 7,8 - 8,1 [2H] s breit.

Die in der folgenden Tabelle aufgeführten Verbindungen (Beispiel 8 bis 54) werden analog hergestellt. Die Identität der Verbindungen wird durch LC-MS nachgewiesen.

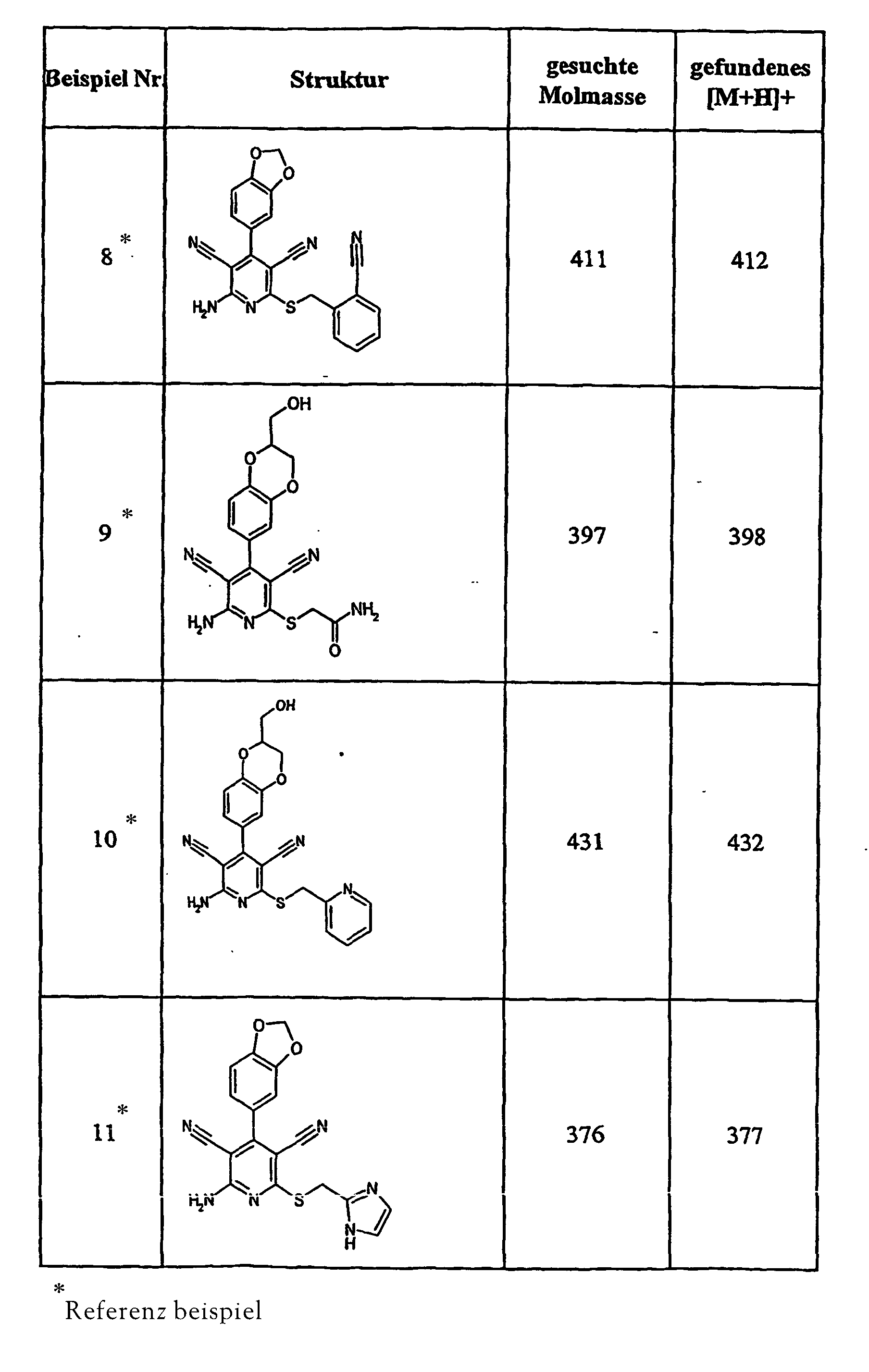

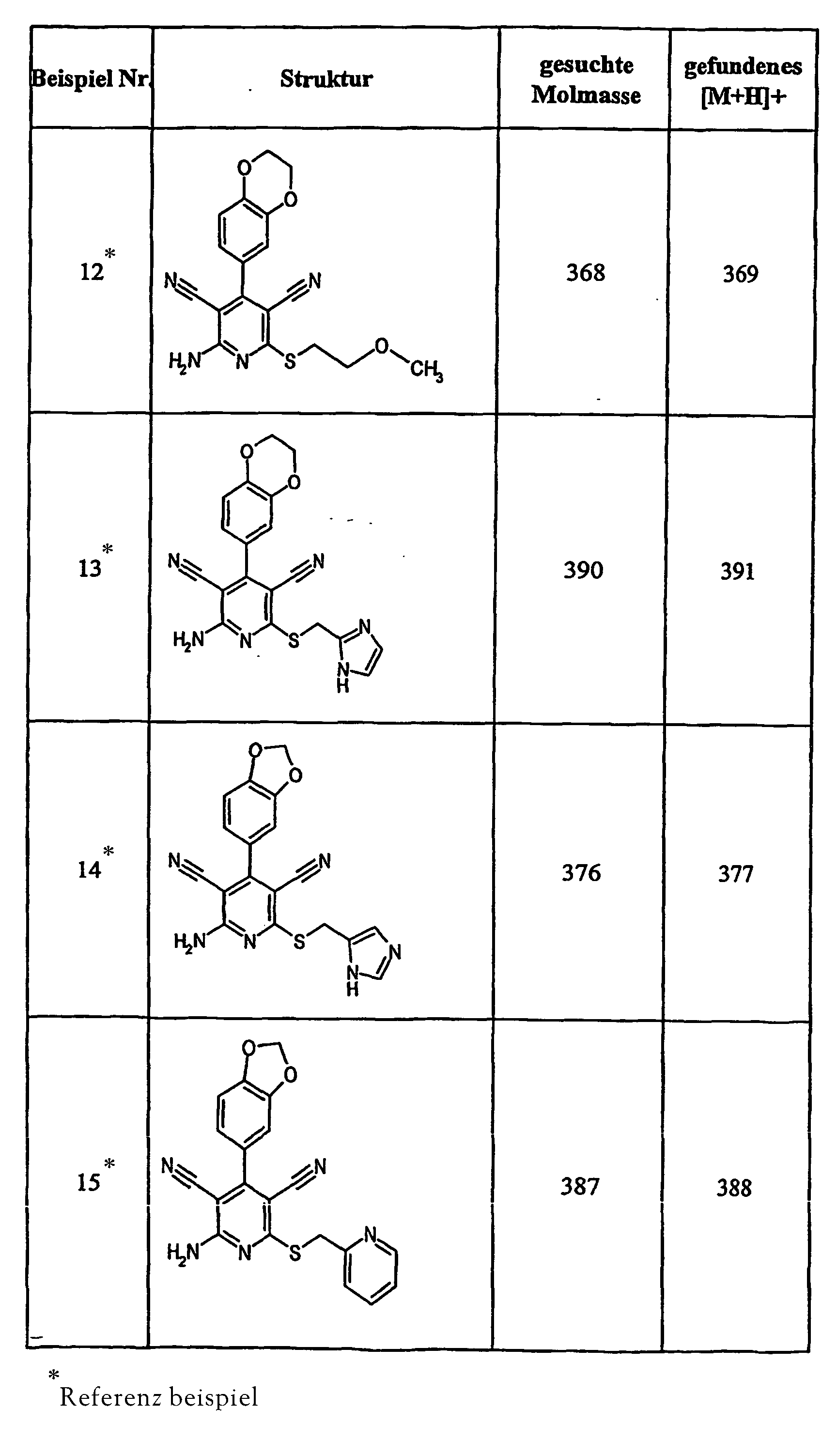

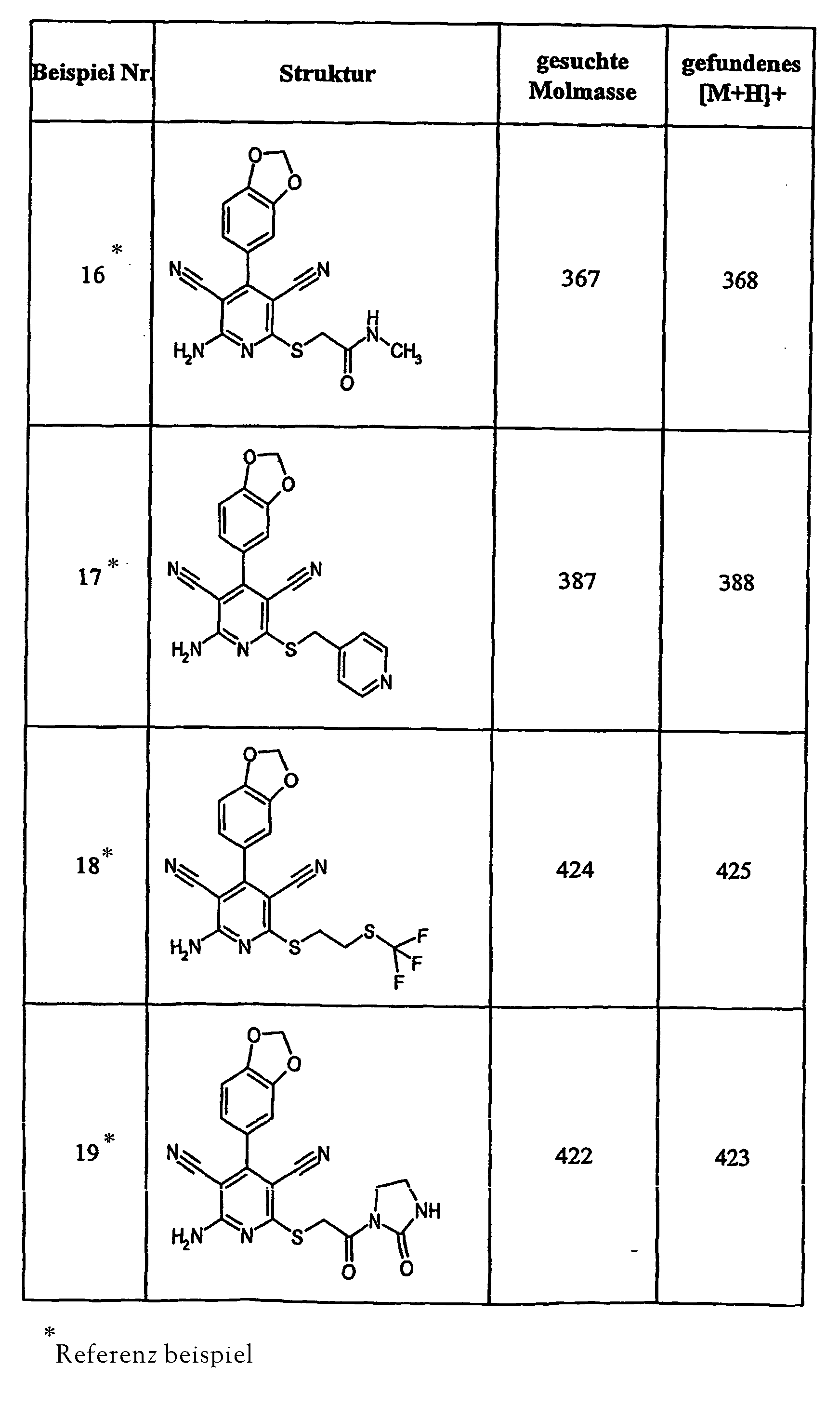

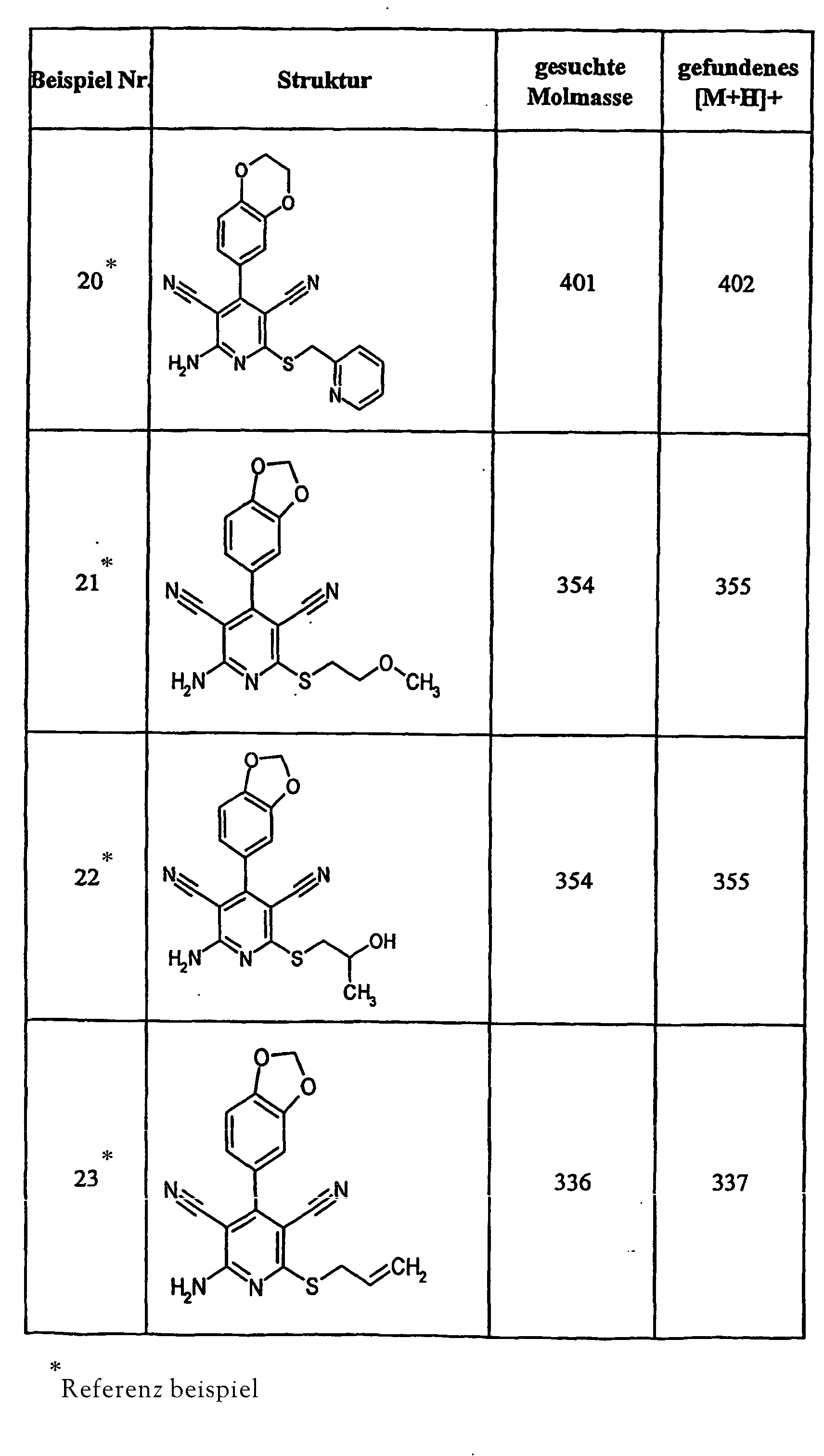

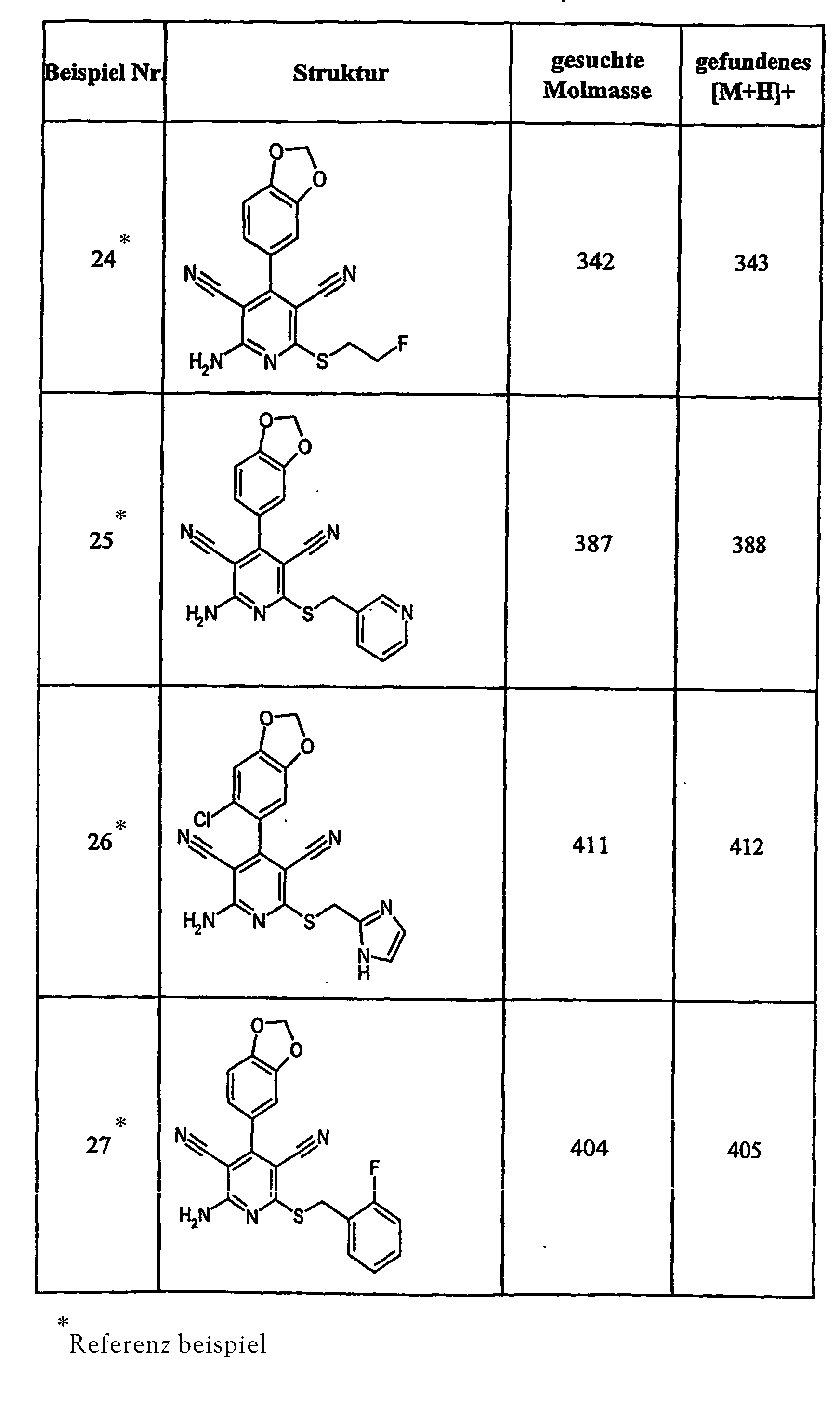

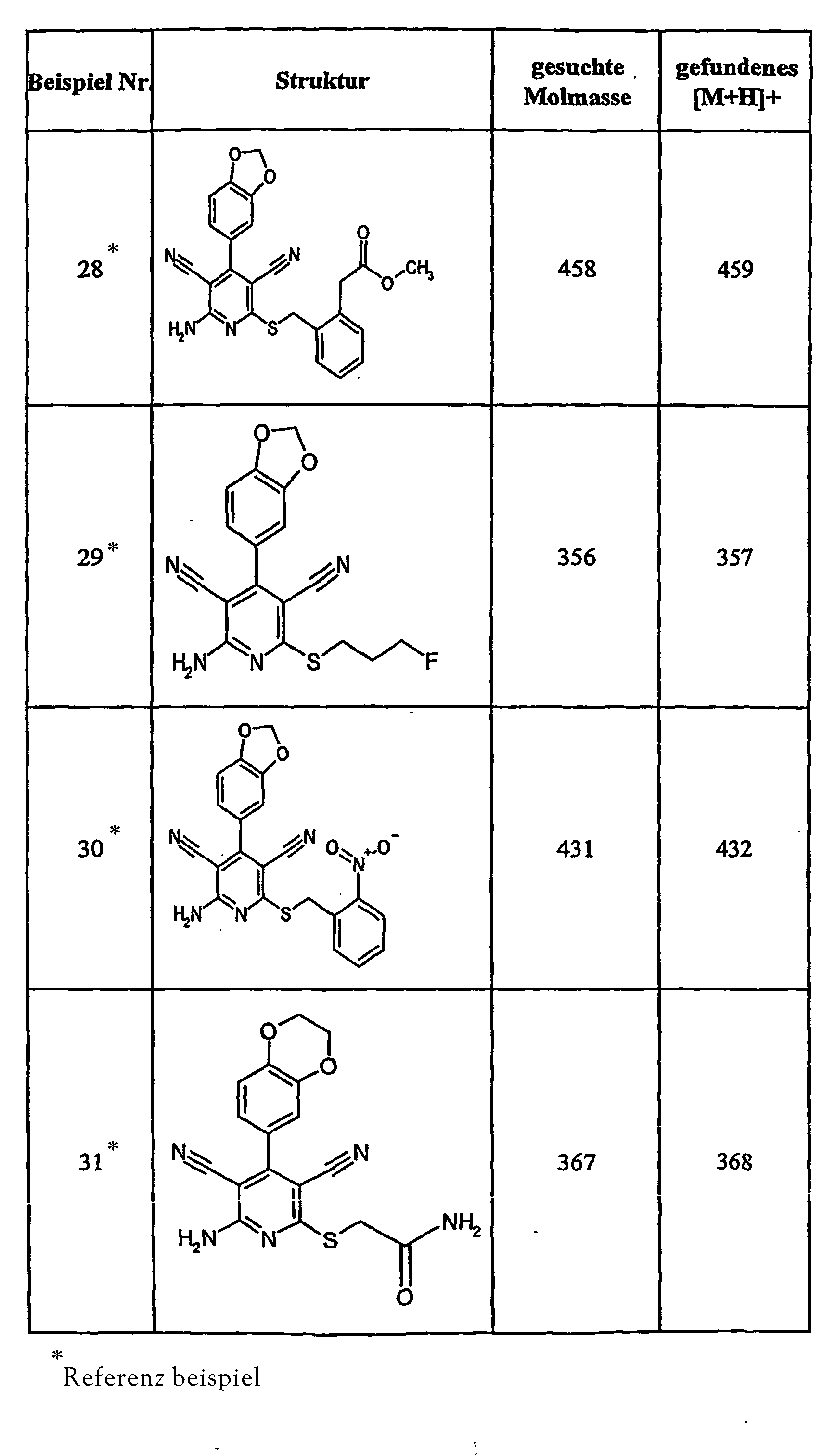

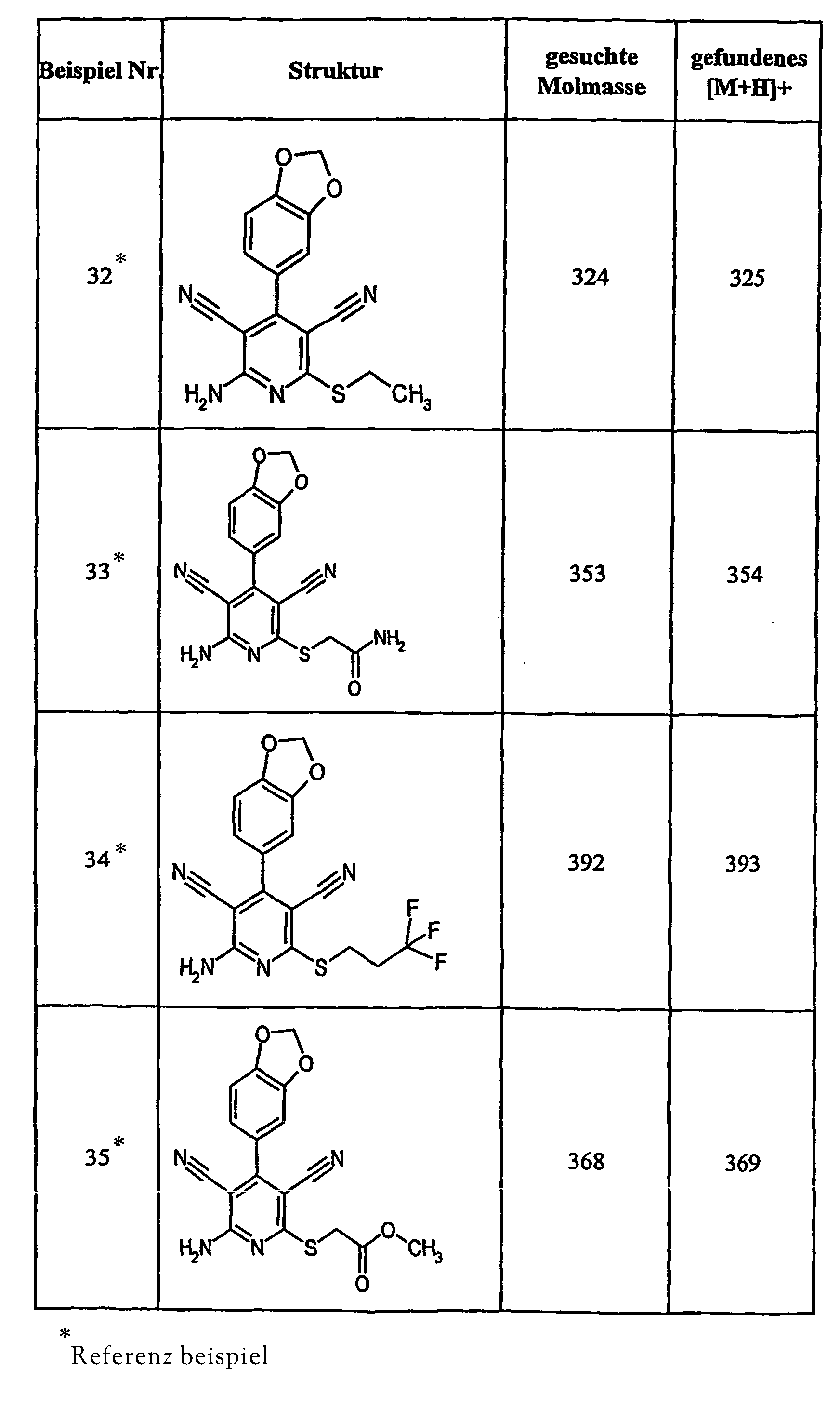

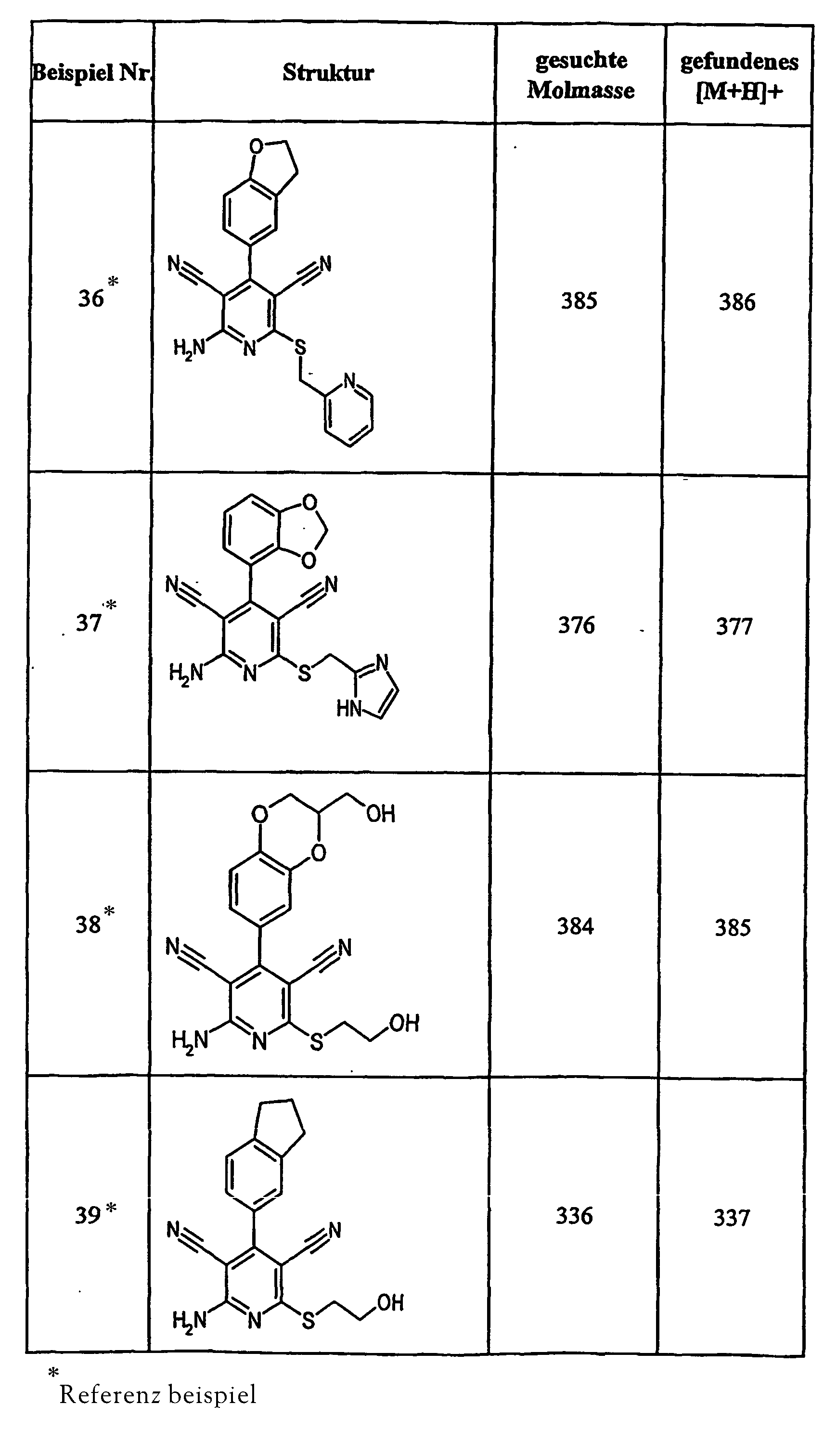

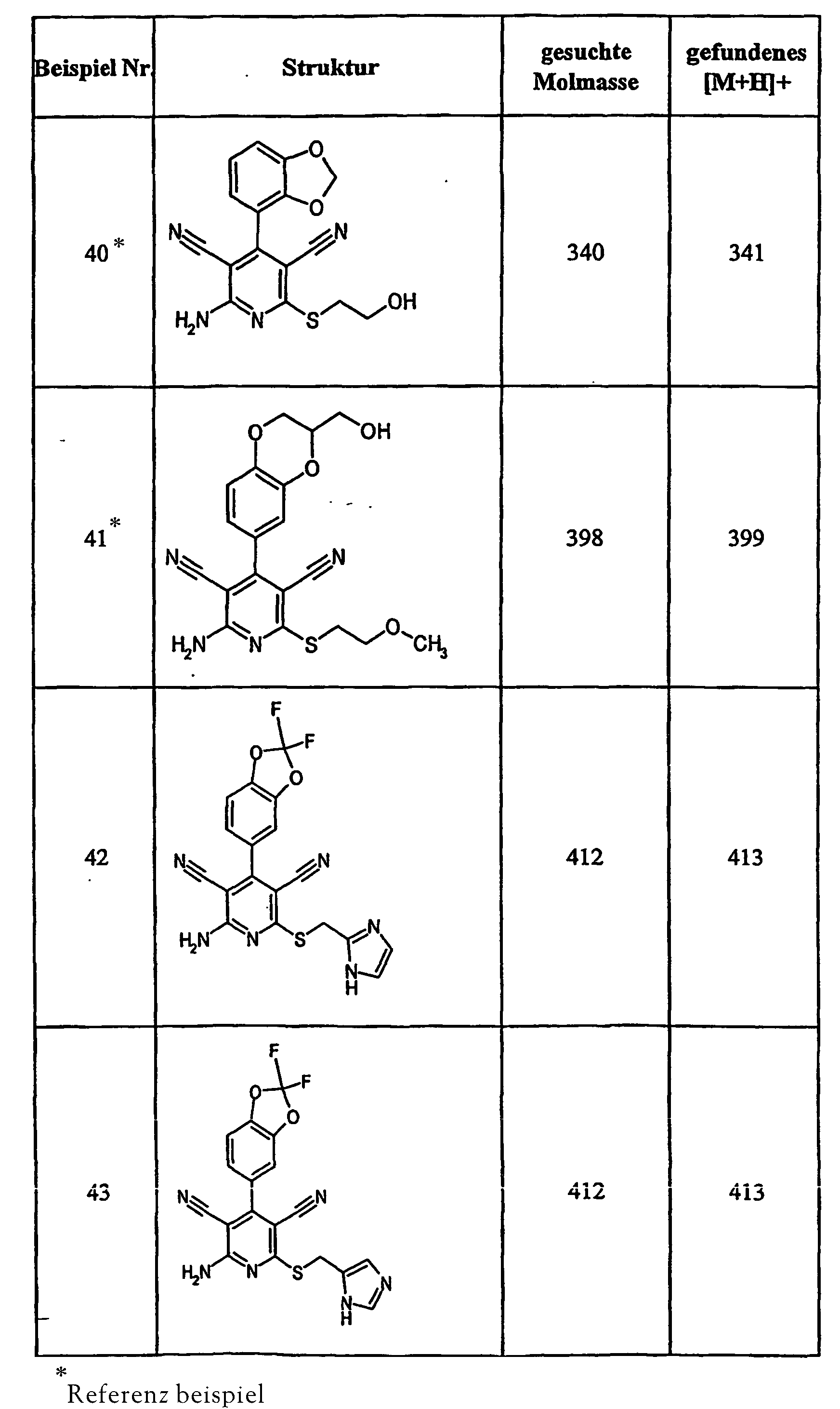

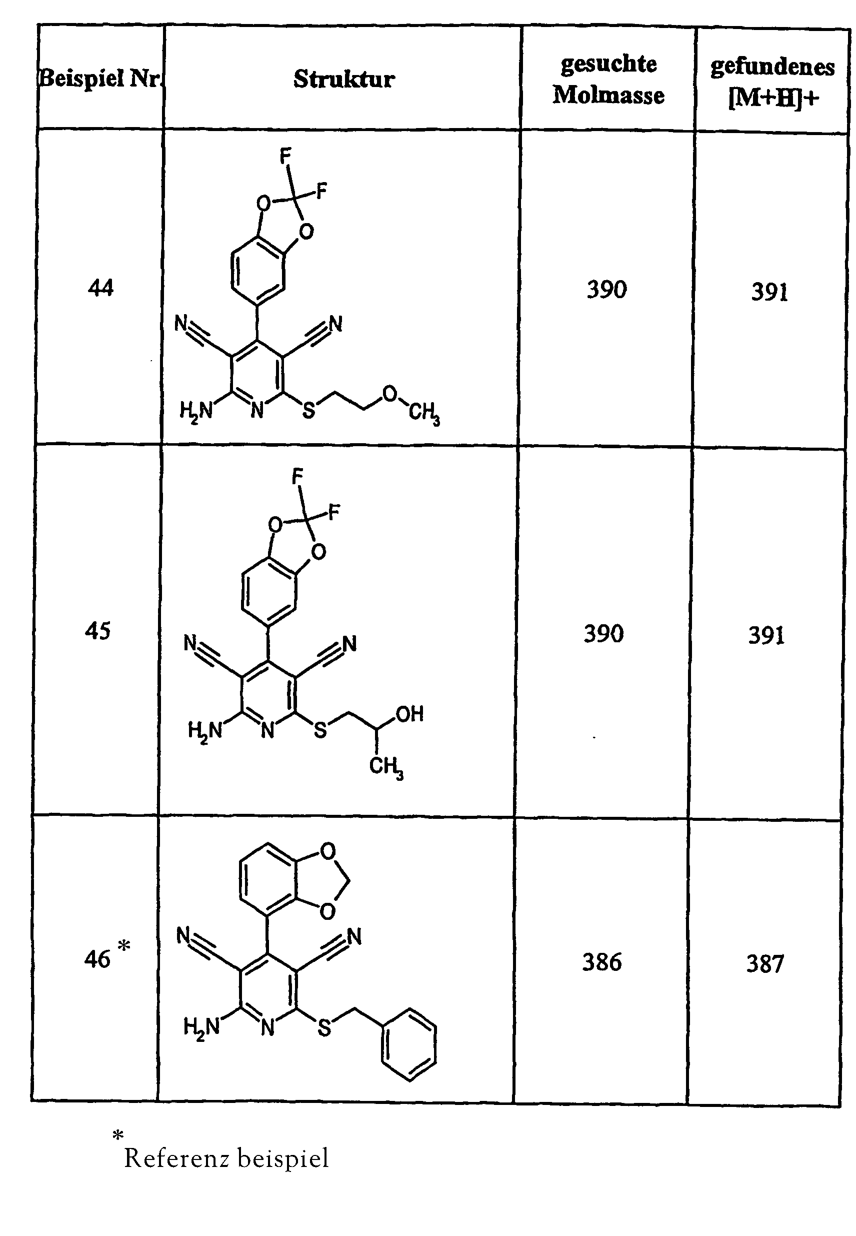

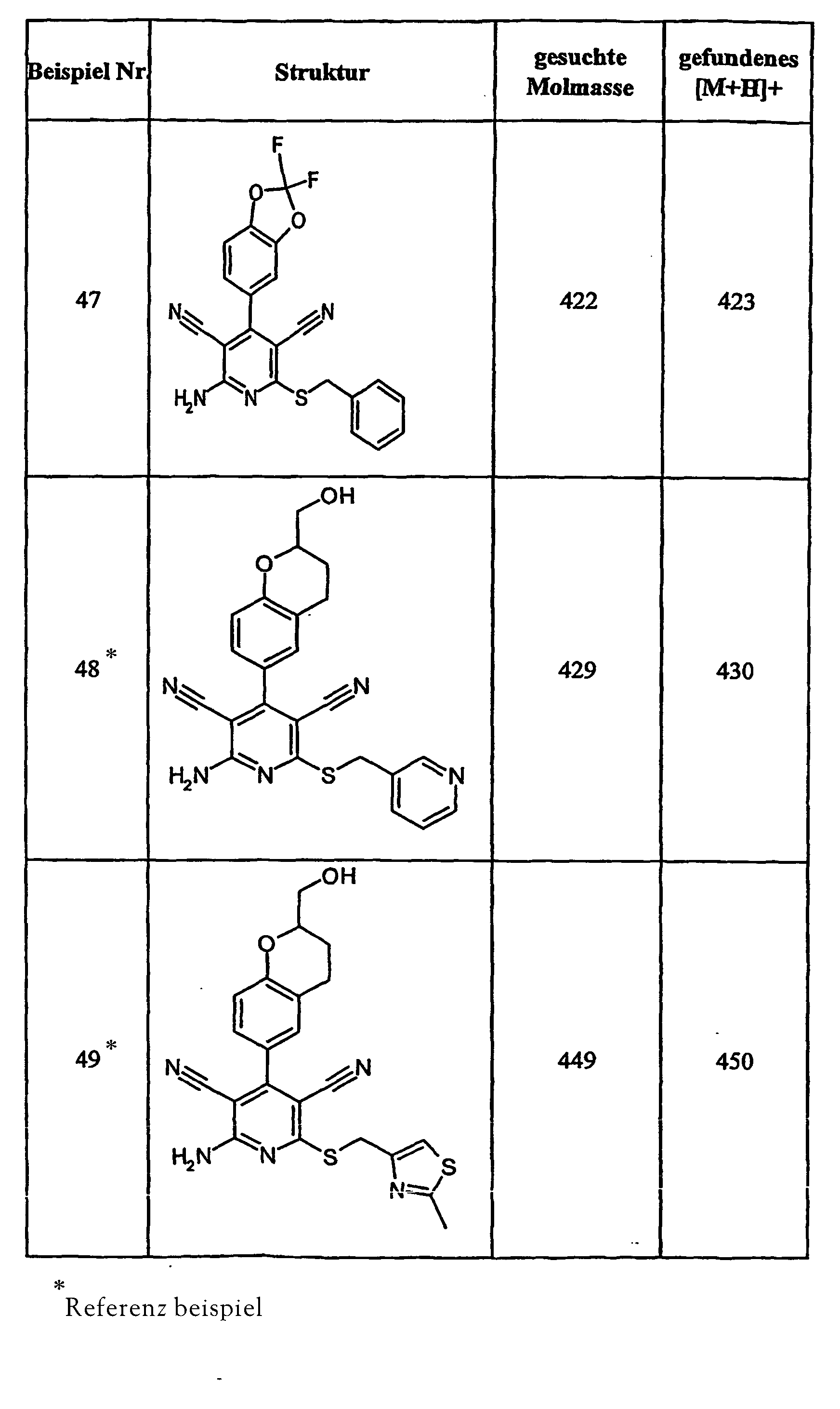

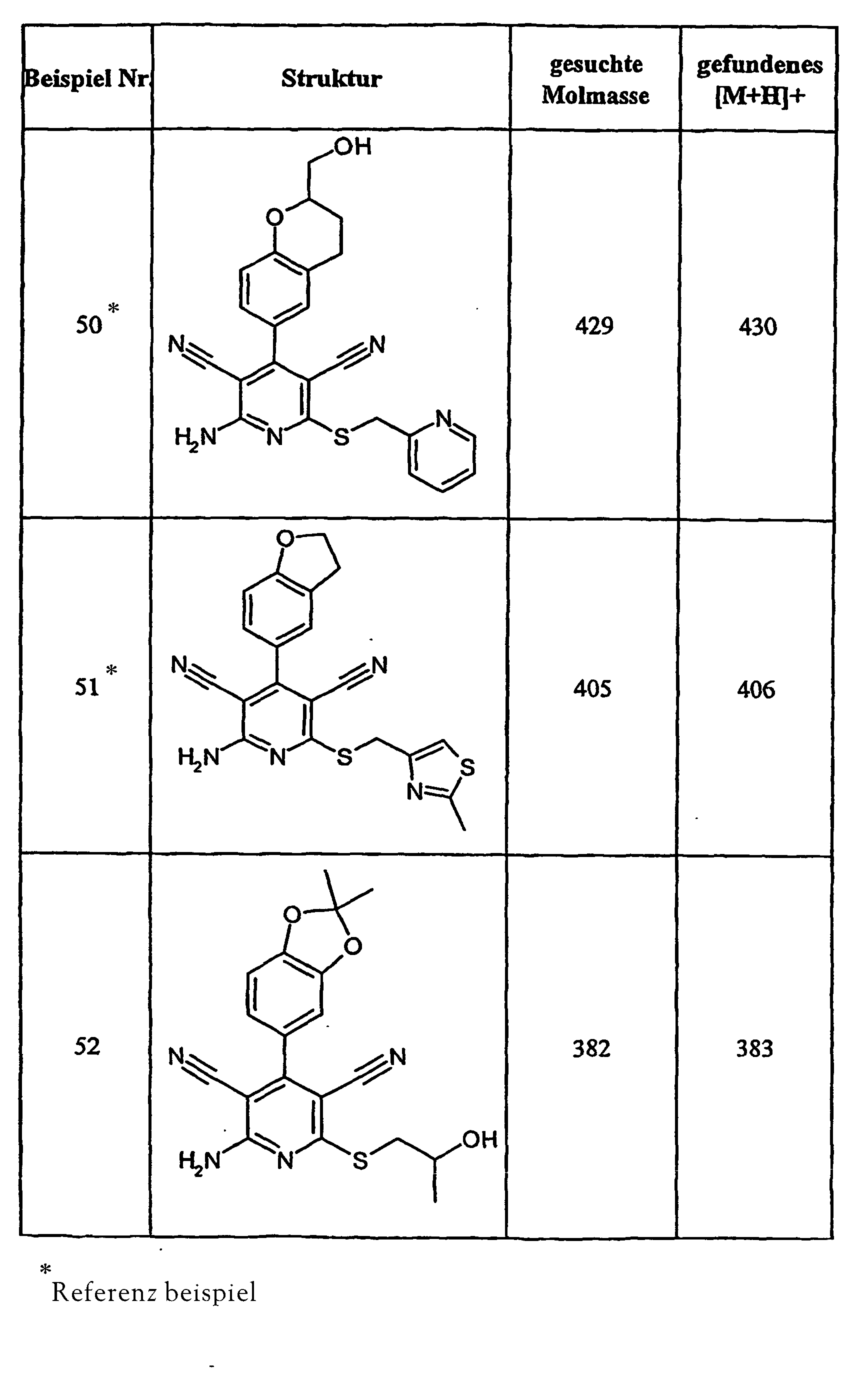

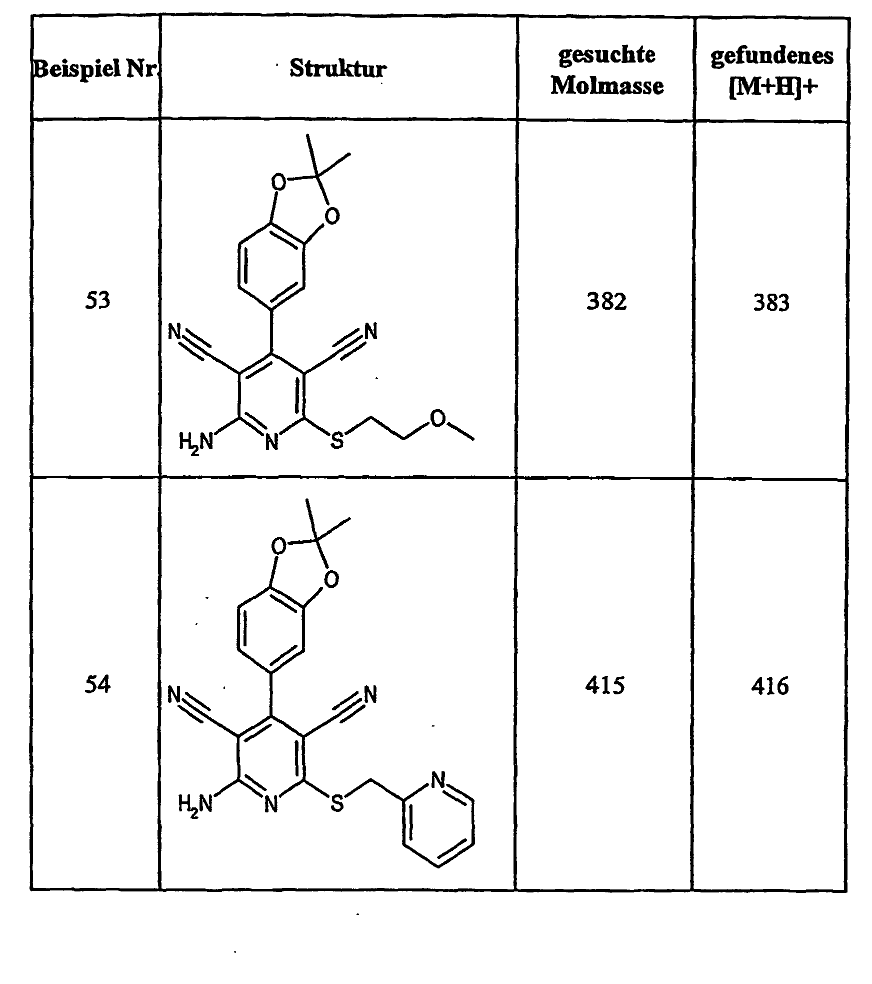

## Patentansprüche

1. Verbindungen der Formel (I) 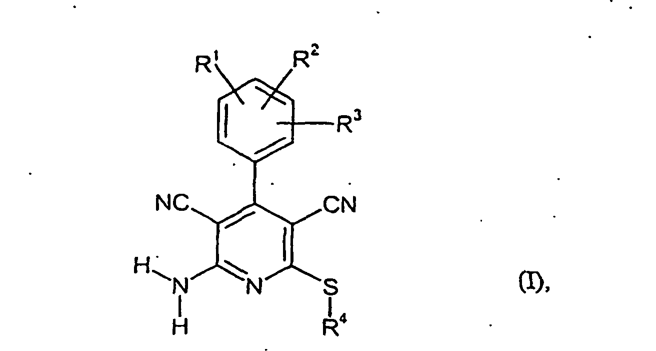 worin
R¹ und R² an benachbarte Phenylringatome gebunden sind und für eine Gruppe 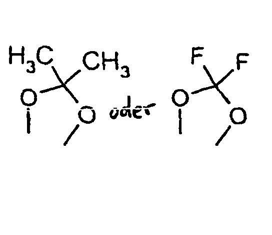 stehen,
R³ Wasserstoff bedeutet,
und
R⁴ Propenyl, Methyl, Ethyl oder n-Propyl bedeutet, wobei die Alkylreste ihrerseits bis zu zweifach, unabhängig voneinander, durch Hydroxy, Methoxy, Trifluormethyl, Trifluormethylthio, Fluor, Imidazolyl, gegebenenfalls durch Methyl substituiertes Thiazolyl, Pyridyl, Phenyl, das seinerseits wiederum durch Fluor, Cyano, Nitro, Methoxy, Methoxycarbonyl (-C(O)-O-CH₃) oder Methoxycarbonylmethyl (-CH₂-C(O)-O-CH₃) substituiert sein kann, Methoxycarbonyl (-C(O)-O-CH₃), Amido (-C(O)-NH₂) oder N-Methylamido (-C(O)-NH-CH₃) substituiert sein können,
und ihre Salze, Hydrate, Hydrate der Salze und Solvate.

2. Verbindungen nach Anspruch 1,
worin
R¹ und R² an benachbarte Phenylringatome gebunden sind und für eine Gruppe 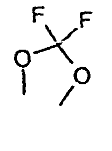 oder 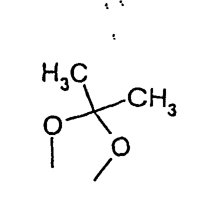 stehen,
R³ Wasserstoff bedeutet,
und
R⁴ Methyl, Ethyl oder n-Propyl bedeutet, wobei die Alkylreste ihrerseits bis zu zweifach, unabhängig voneinander, durch Hydroxy, Trifluormethyl, Trifluormethylthio, Fluor, Imidazolyl, gegebenenfalls durch Methyl substituiertes Thiazolyl, Phenyl, das seinerseits wiederum durch Cyano, Nitro, Methoxycarbonyl (-C(O)-O-CH₃) oder Methoxycarbonylmethyl (-CH₂-C(O)-O-CH₃) substituiert ist, oder Amido (-C(O)-NH₂) substituiert sein können,
und ihre Salze, Hydrate, Hydrate der Salze und Solvate.

3. Verfahren zur Herstellung von Verbindungen der Formel (I), wie in Anspruch 1 definiert, **dadurch gekennzeichnet, dass** man
Verbindungen der Formel (II) 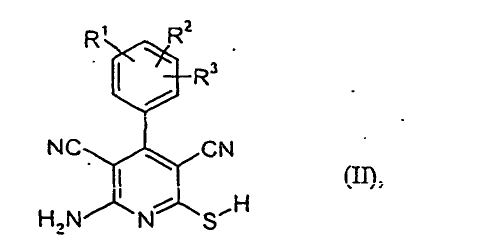 in welcher
die Reste R¹, R² und R³ die in Anspruch 1 angegebene Bedeutung haben,
mit Verbindungen der Formel (III)
R⁴-X (III),
in welcher
R⁴ die in Anspruch 1 angegebene Bedeutung hat und
X für eine Abgangsgruppe steht,
umsetzt.

4. Verbindungen der Formel (I), wie in Anspruch 1 definiert, zur Prophylaxe und/oder Behandlung von Erkrankungen.

5. Zusammensetzung, enthaltend mindestens eine Verbindungen der Formel (I), wie in Anspruch 1 definiert, und mindestens einen weiteren Hilfsstoff.

6. Verwendung von Verbindungen der Formel (I), wie in Anspruch 1 definiert, zur Herstellung von Arzneimitteln zur Prophylaxe und/oder Behandlung von Erkrankungen des Herzkreislaufsystems (kardiovaskulären Erkrankungen).

7. Verwendung von Verbindungen der Formel (I), wie in Anspruch 1 definiert, zur Herstellung von Arzneimitteln zur Prophylaxe und/oder Behandlung von Erkrankungen des Urogenitalbereichs und Krebs.

8. Verwendung von Verbindungen der Formel (I), wie in Anspruch 1 definiert, zur Herstellung von Arzneimitteln zur Prophylaxe und/oder Behandlung von inflammatorischen und neuroinflammatorischen Erkrankungen, neurodegenerativen Erkrankungen und Schmerzzuständen.

9. Verwendung von Verbindungen der Formel (I), wie in Anspruch 1 definiert, zur Herstellung von Arzneimitteln zur Prophylaxe und/oder Behandlung von Erkrankungen der Atemwege, von Leberfibrose und Leberzirrhose und Diabetes.

## Claims

1. Compounds of the formula (I) 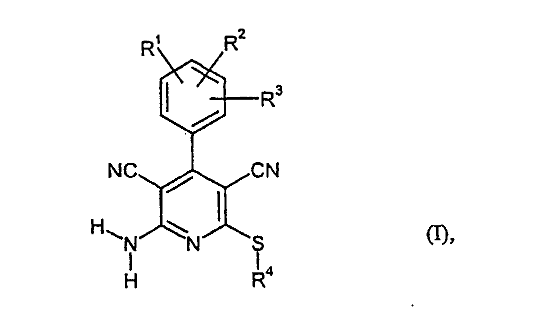 in which
R¹ and R² are attached to adjacent phenyl ring atoms and represent a group 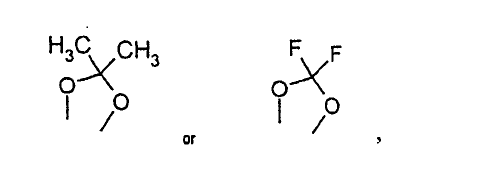
R³ x represents hydrogen
and
R⁴ represents propenyl, methyl, ethyl or n-propyl, where the alkyl radicals for their part may be substituted up to two times, independently of one another, by hydroxyl, methoxy, trifluoromethyl, trifluoromethylthio, fluorine, imidazolyl, optionally methyl-substituted thiazolyl, pyridyl, phenyl, which for its part may be substituted by fluorine, cyano, nitro, methoxy, methoxycarbonyl (-C(O)-O-CH₃) or methoxycarbonylmethyl (-CH₂-C(O)-O-CH₃), methoxycarbonyl (-C(O)-O-CH₃), amido (-C(O)-NH₂) or N-methylamido (-C(O)-NH-CH₃),
and the salts, hydrates, hydrates of the salts and solvates thereof.

2. Compounds according to Claim 1
in which
R¹ and R² are attached to adjacent phenyl ring atoms and represent a group 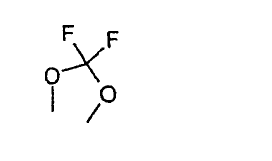 or 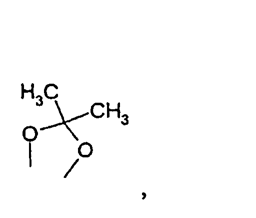
R³ represents hydrogen
and
R⁴ represents methyl, ethyl or n-propyl, where the alkyl radicals for their part may be substituted up to two times, independently of one another, by hydroxyl, trifluoromethyl, trifluoromethylthio, fluorine, imidazolyl, optionally methyl-substituted thiazolyl, phenyl which for its part is substituted by cyano, nitro, methoxycarbonyl (-C(O)-O-CH₃) or methoxycarbonylmethyl (-CH₂-C(O)-O-CH₃), or amido (-C(O)-N H₂),
and the salts, hydrates, hydrates of the salts and solvates thereof.

3. Process for preparing compounds of the formula (I) as defined in Claim 1, **characterized in that**
compounds of the formula (II) 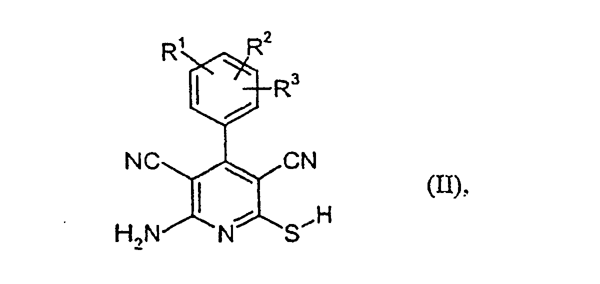 in which
the radicals R¹, R² and R³ are as defined in Claim 1,
are reacted with compounds of the formula (III)
R⁴-X (III)
in which
R⁴ is as defined in Claim 1 and
X represents a leaving group.

4. Compounds of the formula (I) as defined in Claim 1 for the prophylaxis and/or treatment of disorders.

5. Composition comprising at least one compound of the formula (I) as defined in Claim 1 and at least one auxiliary.

6. Use of compounds of the formula (I) as defined in Claim 1 for preparing medicaments for the prophylaxis and/or treatment of disorders of the cardiovascular system (cardiovascular disorders).

7. Use of compounds of the formula (I) as defined in Claim 1 for preparing medicaments for the prophylaxis and/or treatment of disorders of the urogenital system and cancer.

8. Use of compounds of the formula (I) as defined in Claim 1 for preparing medicaments for the prophylaxis and/or treatment of inflammatory and neuroinflammatory disorders, neurodegenerative disorders and pain.

9. Use of compounds of the formula (I) as defined in Claim 1 for preparing medicaments for the prophylaxis and/or treatment of disorders of the respiratory tract, of liver fibrosis and liver cirrhosis and diabetes.

## Revendications

1. Composés de formule (I) 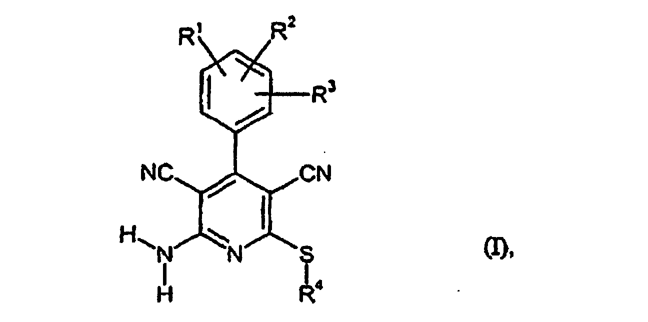 dans laquelle
R¹ et R² sont liés à des atomes contigus du noyau phényle et représentent un groupe 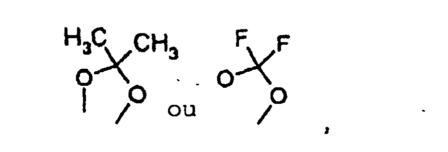 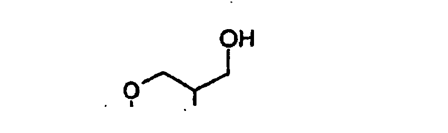
R³ représente l'hydrogène,
et
R⁴ est un reste propényle, méthyle, éthyle ou n-propyle, les restes alkyle pouvant eux-mêmes porter jusqu'à deux substituants, indépendamment l'un de l'autre, hydroxy, méthoxy, trifluorométhyle, trifluorométhylthio, fluoro, imidazolyle, thiazolyle portant éventuellement un substituant méthyle, pyridyle, phényle pouvant lui-même porter un substituant fluoro, cyano, nitro, méthoxy, méthoxycarbonyle (-C(O)-O-CH₃) ou méthoxycarbonylméthyle (-CH₂-C(O)-O-CH₃), méthoxycarbonyle (-C(O)-O-CH₃), amido (-C(O)-O-NH₂) ou N-méthylamido (-C(O)-NH-CH₃),
et leurs sels, leurs hydrates, les hydrates de leurs sels et leurs produits de solvatation.

2. Composés suivant la revendication 1,
dans lesquels
R¹ et R² sont liés à des atomes contigus du noyau phényle et représentent un groupe 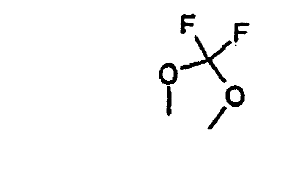 ou 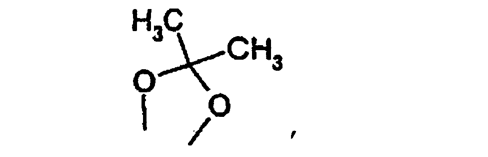
R³ représente l'hydrogène,
et
R⁴ est un reste méthyle, éthyle ou n-propyle, les restes alkyle pouvant eux-mêmes porter jusqu'à deux substituants, indépendants l'un de l'autre, hydroxy, trifluorométhyle, trifluorométhylthio, fluoro, imidazolyle, thiazolyle éventuellement substitué par un radical méthyle, phényle lui-même substitué par un radical cyano, nitro, méthoxycarbonyle (-C(O)-O-CH₃) ou méthoxycarbonylméthyle (-CH₂-C(O)-O-CH₃), ou amido (-C(O)-O-NH₂),
et leurs sels, leurs hydrates, les hydrates de leurs sels et leurs produits de solvatation.

3. Procédé de production de composés de formule (I), tels que définis dans la revendication 1, **caractérisé en ce qu'**on fait réagir des composés de formule (II) 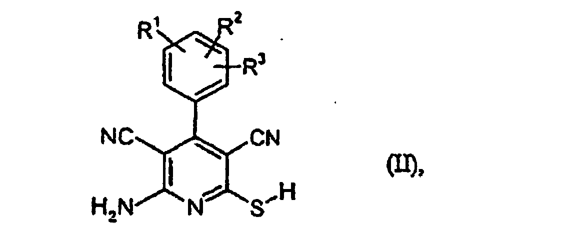 dans laquelle
les restes R¹, R² et R³ ont la définition indiquée dans la revendication 1,
avec des composés de formule (III)
**R**^{**4**}**-X (III),**
dans laquelle
R⁴ a la définition indiquée dans la revendication 1 et
X est un groupe partant.

4. Composés de formule (I), tels que définis dans la revendication 1, destinés à la prophylaxie et/ou au traitement de maladies.

5. Composition contenant au moins un composé de formule (I), tel que défini dans la revendication 1, et au moins une autre substance auxiliaire.

6. Utilisation de composés de formule (I), tels que définis dans la revendication 1, pour la préparation de médicaments destinés à la prophylaxie et/ou au traitement de maladies du système cardio-vasculaire (maladies cardio-vasculaires).

7. Utilisation de composés de formule (I), tels que définis dans la revendication 1, pour la préparation de médicaments destinés à la prophylaxie et/ou au traitement de maladies du système urogénital et du cancer.

8. Utilisation de composés de formule (I), tels que définis dans la revendication 1, pour la préparation de médicaments destinés à la prophylaxie et/ou au traitement de maladies inflammatoires et neuro-inflammatoires, de maladies neurodégénératives et d'états douloureux.

9. Utilisation de composés de formule (I), tels que définis dans la revendication 1, pour la préparation de médicaments destinés à la prophylaxie et/ou au traitement d'affections des voies respiratoires, de la fibrose du foie et de la cirrhose du foie, et du diabète.
